# EUROPEAN PATENT APPLICATION

(11) **EP 4 339 207 A1**
(43) Date of publication of application: **20.03.2024**
(21) Application number: 22806818.5
(22) Date of filing: 12.05.2022
(51) Int. Cl.: C07K 16/28, A61K 39/395, A61P 35/00, A61K 45/00

(54) **ANTIGEN BINDING MOLECULE SPECIFICALLY BINDING TO RANKL AND NGF, AND MEDICAL USE THEREOF**

(30) Priority: 12.05.2021 CN 202110515444
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YING, Hua, Shanghai 200245 (CN); MAO, Langyong, Shanghai 200245 (CN); GU, Xiaoling, Shanghai 200245 (CN); TAO, Weikang, Shanghai 200245 (CN)
(74) Representative: Dragotti & Associati S.R.L.
(86) International application number: PCT/CN2022/092333
(87) International publication number: WO 2022/237856

(57) **Abstract**

The present disclosure relates to an antigen-binding molecule specifically binding to RANKL and NGF, and a medical use thereof. Specifically, provided are a novel anti-RANKL antibody, a bispecific antibody targeting RANKL and NGF, a preparation method for the antibody, and a use thereof in treatment or prevention of diseases.

## Description

The present application claims priority to the Chinese Patent Application (Application No. 202110515444.9) filed on May 12, 2021.

### TECHNICAL FIELD

The present disclosure belongs to the field of biopharmaceuticals. More specifically, the present disclosure relates to antigen-binding molecules that specifically bind to RANKL and NGF and use thereof.

### BACKGROUND

The statements herein merely provide background information related to the present disclosure and may not necessarily constitute the prior art.

Bone metastasis is a common accompanying symptom in later stages of many cancers. The pathogenesis of bone metastasis: Primary tumor cells fall off and enter the blood circulation system, forming circulating tumor cells, and the circulating tumor cells are transferred to a bone part, form transmissible tumor cells and lie dormant in a bone microenvironment. This process may continue for years until the surviving tumor cells gradually adapt to the bone microenvironment. The tumor cells are activated, transitioning from the dormant state to a proliferative state (Mantyh, P.W., Curr Opin Support Palliat Care, 2014. 8(2):p. 83-90). The proliferation of the tumor cells affects the bone microenvironment and promotes osteoclast formation, which leads to accelerated bone resorption and release of various cytokines, further promoting tumor cell proliferation. Hence, a vicious cycle is formed (Quayle, L., Curr Cancer Drug Targets, 2015. 15(6):p. 469-480). The constant proliferation of tumor cells leads to the formation of multiple metastatic sites, which causes various problems such as bone injury and pain in patients (Gartland, A., J Bone Oncol, 2016. 5(3): p. 100-103).

According to statistics, common cancers involving bone metastasis include myeloma, kidney cancer, melanoma, bladder cancer, thyroid cancer, lung cancer, breast cancer and prostate cancer (Clezardin, P., Joint Bone Spine, 2017. 84(6): p. 677-684. Fidler, M.M., Scand J Public Health, 2018. 46(1):p. 27-36).

In normal bone tissue, osteoblasts and osteoclasts are in a relatively balanced state to maintain the normal growth and development of bones. However, the presence of cancer cells will break the balance. Cancer cells in bone microenvironments release a series of cytokines to stimulate the secretion of a large amount of RANKL (receptor activator of nuclear factor-κB ligand) from osteoblasts and osteocytes. The interaction of RANKL with the receptor RANK promotes osteoclast formation, leading to increased bone resorption. Osteolysis caused by bone resorption causes the release of other growth factors to promote cancer cell proliferation. Hence, a cycle that favors the metastasis of cancer cells is formed (Body, J.J., Expert Rev Anticancer Ther, 2012. 12(3):p. 307-322).

NGF is a nerve growth factor. The NGF signaling pathway mediates the growth and development of the nervous system and the transmission of pain signals. At present, there are two known receptors for NGF on the cell surface: the high-affinity receptor TrKA and the low-affinity receptor p75NTR. The interaction of NGF with TrkA activates the Ras and PI3K pathways simultaneously, promoting cell survival and nerve growth. The interaction of NGF with p75NTR promotes apoptosis. Moreover, the PI3K pathway also activates the phosphorylation of TRPV1, causing ion channels to be activated to generate action potentials and transmit signals from pain neurons (Kumar, V. and B.A. J Pain Res, 2012. 5:p. 279-287).

Monoclonal antibodies targeting NGF and RANKL have been shown to have certain therapeutic effects on bone injury and pain caused by bone metastasis (Body JJ. Expert Rev Anticancer Ther. 2012. 12(3):p.307-322. Sopata M, et al. 2015. 156(9):p. 1703-1713).

### SUMMARY

The present disclosure provides a bispecific antibody that blocks RANKL and NGF simultaneously and thus the two critical signaling pathways associated with bone loss and pain; in addition, the bispecific antibody has good analgesic and bone protection effects *in vivo* and has good safety.

In one aspect, the present disclosure provides an antigen-binding molecule comprising at least one first antigen-binding domain that specifically binds to RANKL and at least one second antigen-binding domain that specifically binds to NGF.

In some embodiments, in the aforementioned antigen-binding molecule, the first antigen-binding domain that specifically binds to RANKL comprises a heavy chain variable region and a light chain variable region, wherein:
i) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
ii) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 15; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
iii) the heavy chain variable region comprises: a HCDR1 comprising the amino acid sequence of SEQ ID NO: 13; a HCDR2 comprising the amino acid sequence of SEQ ID NO: 8; and a HCDR3 comprising the amino acid sequence of SEQ ID NO: 3; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6.

In some embodiments, in the aforementioned antigen-binding molecule, the first antigen-binding domain that specifically binds to RANKL comprises a heavy chain variable region and a light chain variable region, wherein:
i) the heavy chain variable region comprises a HCDR1, a HCDR2 and a HCDR3 set forth in SEQ ID NO: 7, SEQ ID NO: 8 and SEQ ID NO: 14, respectively; and
   the light chain variable region comprises a LCDR1, a LCDR2 and a LCDR3 set forth in SEQ ID NO: 4, SEQ ID NO: 5 and SEQ ID NO: 6, respectively; or
ii) the heavy chain variable region comprises a HCDR1, a HCDR2 and a HCDR3 set forth in SEQ ID NO: 7, SEQ ID NO: 8 and SEQ ID NO: 15, respectively; and
   the light chain variable region comprises a LCDR1, a LCDR2 and a LCDR3 set forth in SEQ ID NO: 4, SEQ ID NO: 5 and SEQ ID NO: 6, respectively; or
iii) the heavy chain variable region comprises a HCDR1, a HCDR2 and a HCDR3 set forth in SEQ ID NO: 13, SEQ ID NO: 8 and SEQ ID NO: 3, respectively; and
   the light chain variable region comprises a LCDR1, a LCDR2 and a LCDR3 set forth in SEQ ID NO: 4, SEQ ID NO: 5 and SEQ ID NO: 6, respectively.

In some embodiments, in the aforementioned antigen-binding molecule, the first antigen-binding domain that specifically binds to RANKL comprises a heavy chain variable region and a light chain variable region, wherein:
the heavy chain variable region comprises an amino acid sequence having at least 90%, 91%, 92%, 93%, 94%, 95%, 96%, 97%, 98% or 99% sequence identity to SEQ ID NO: 36, 37 or 38; and/or
the light chain variable region comprises an amino acid sequence having at least 90%, 91%, 92%, 93%, 94%, 95%, 96%, 97%, 98% or 99% sequence identity to SEQ ID NO: 40.

In some embodiments, in the aforementioned antigen-binding molecule, the first antigen-binding domain that specifically binds to RANKL comprises a heavy chain variable region and a light chain variable region, wherein the heavy chain variable region comprises an amino acid sequence having at least 90%, 95%, 96%, 97%, 98% or 99% sequence identity to SEQ ID NO: 37; and/or
the light chain variable region comprises an amino acid sequence having at least 90%, 91%, 92%, 93%, 94%, 95%, 96%, 97%, 98% or 99% identity to SEQ ID NO: 40.

In some embodiments, in the aforementioned antigen-binding molecule, the first antigen-binding domain that specifically binds to RANKL comprises a heavy chain variable region and a light chain variable region, wherein the heavy chain variable region comprises the amino acid sequence of SEQ ID NO: 36, 37 or 38; and/or the light chain variable region comprises the amino acid sequence of SEQ ID NO: 40.

In some embodiments, in the aforementioned antigen-binding molecule, the first antigen-binding domain that specifically binds to RANKL comprises a heavy chain variable region and a light chain variable region, wherein the sequence of the heavy chain variable region is set forth in SEQ ID NO: 36, 37 or 38; and/or the sequence of the light chain variable region is set forth in SEQ ID NO: 40.

In some embodiments, in the aforementioned antigen-binding molecule, the first antigen-binding domain that specifically binds to RANKL comprises a heavy chain variable region and a light chain variable region, wherein the heavy chain variable region comprises the amino acid sequence of SEQ ID NO: 37; and/or the light chain variable region comprises the amino acid sequence of SEQ ID NO: 40.

In some embodiments, in the aforementioned antigen-binding molecule, the first antigen-binding domain that specifically binds to RANKL comprises a heavy chain variable region and a light chain variable region, wherein the heavy chain variable region comprises the amino acid sequence of SEQ ID NO: 36; and/or the light chain variable region comprises the amino acid sequence of SEQ ID NO: 40.

In some embodiments, in the aforementioned antigen-binding molecule, the first antigen-binding domain that specifically binds to RANKL comprises a heavy chain variable region and a light chain variable region, wherein the heavy chain variable region comprises the amino acid sequence of SEQ ID NO: 38; and/or the light chain variable region comprises the amino acid sequence of SEQ ID NO: 40.

In some embodiments, in the aforementioned antigen-binding molecule, the first antigen-binding domain that specifically binds to RANKL comprises a heavy chain variable region and a light chain variable region, wherein the sequence of the heavy chain variable region is set forth in SEQ ID NO: 37; and/or the sequence of the light chain variable region is set forth in SEQ ID NO: 40.

In some embodiments, in the aforementioned antigen-binding molecule, the second antigen-binding domain that specifically binds to NGF comprises a heavy chain variable region and a light chain variable region, wherein:
the heavy chain variable region comprises: a HCDR1 comprising the amino acid sequence of SEQ ID NO: 58; a HCDR2 comprising the amino acid sequence of SEQ ID NO: 59; and a HCDR3 comprising the amino acid sequence of SEQ ID NO: 60; and
the light chain variable region comprises: a LCDR1 comprising the amino acid sequence of SEQ ID NO: 61; a LCDR2 comprising the amino acid sequence of SEQ ID NO: 62; and a LCDR3 comprising the amino acid sequence of SEQ ID NO: 63.

In some embodiments, in the aforementioned antigen-binding molecule, the second antigen-binding domain that specifically binds to NGF comprises a heavy chain variable region and a light chain variable region, wherein:
the heavy chain variable region comprises a HCDR1, a HCDR2 and a HCDR3 set forth in SEQ ID NO: 58, SEQ ID NO: 59 and SEQ ID NO: 60, respectively; and
the light chain variable region comprises a LCDR1, a LCDR2 and a LCDR3 set forth in SEQ ID NO: 61, SEQ ID NO: 62 and SEQ ID NO: 63, respectively.

In some embodiments, in the aforementioned antigen-binding molecule, the second antigen-binding domain that specifically binds to NGF comprises a heavy chain variable region and a light chain variable region, wherein:
the heavy chain variable region comprises an amino acid sequence having at least 90%, 91%, 92%, 93%, 94%, 95%, 96%, 97%, 98% or 99% sequence identity to SEQ ID NO: 64; and/or the light chain variable region comprises an amino acid sequence having at least 90%, 91%, 92%, 93%, 94%, 95%, 96%, 97%, 98% or 99% sequence identity to SEQ ID NO: 65.

In some embodiments, in the aforementioned antigen-binding molecule, the second antigen-binding domain that specifically binds to NGF comprises a heavy chain variable region and a light chain variable region, wherein the heavy chain variable region comprises the amino acid sequence of SEQ ID NO: 64; and/or the light chain variable region comprises the amino acid sequence of SEQ ID NO: 65.

In some embodiments, in the aforementioned antigen-binding molecule, the second antigen-binding domain that specifically binds to NGF comprises a heavy chain variable region and a light chain variable region, wherein the sequence of the heavy chain variable region is set forth in SEQ ID NO: 64; and/or the sequence of the light chain variable region is set forth in SEQ ID NO: 65.

In some embodiments, the aforementioned antigen-binding molecule comprises an Fc region comprising two subunits capable of associating (dimerizing).

In some embodiments, in the aforementioned antigen-binding molecule, the Fc region is an IgG₁, IgG₂, IgG₃ or IgG₄ Fc region; preferably, the Fc region is an IgG4 Fc region.

In some embodiments, in the aforementioned antigen-binding molecule, the Fc region comprises one or more amino acid substitutions.

In some embodiments, in the aforementioned antigen-binding molecule, the Fc region is a human IgG₄ Fc region, and the amino acid residue at position 228 is P, as numbered according to the EU index.

In some embodiments, the aforementioned antigen-binding molecule comprises a first chain of the structure of formula (I) and a second chain of the structure of formula (II),

formula (I): VHa-linker 1-VHb-CH1-one subunit of Fc region;

formula (II): VLa-linker 2-VLb-CL;

wherein: VHa and VHb are heavy chain variable regions, and VLa and VLb are light chain variable regions; and
VHa and VLa can form the first antigen-binding domain that specifically binds to RANKL, and VHb and VLb can form the second antigen-binding domain that specifically binds to NGF; or
VHa and VLa can form the second antigen-binding domain that specifically binds to NGF, and VHb and VLb can form the first antigen-binding domain that specifically binds to RANKL; and
linker 1 and linker 2 are peptide linkers identical or different in sequence.

In some embodiments, the aforementioned antigen-binding molecule comprises two first chains of the structure of formula (I) and two second chains of the structure of formula (II),

formula (I): VHa-linker 1-VHb-CH1-one subunit of Fc region;

formula (II): VLa-linker 2-VLb-CL;

wherein: VHa and VHb are heavy chain variable regions, and VLa and VLb are light chain variable regions; and
VHa and VLa can form the first antigen-binding domain that specifically binds to RANKL, and VHb and VLb can form the second antigen-binding domain that specifically binds to NGF; or
VHa and VLa can form the second antigen-binding domain that specifically binds to NGF, and VHb and VLb can form the first antigen-binding domain that specifically binds to RANKL; and
linker 1 and linker 2 are peptide linkers identical or different in sequence.

In some embodiments, the aforementioned antigen-binding molecule comprises a first chain of the structure of formula (I) and a second chain of the structure of formula (II),

formula (I): VHa-linker 1-VHb-CH1-one subunit of Fc region;

formula (II): VLa-linker 2-VLb-CL;

wherein: VHa and VHb are heavy chain variable regions, and VLa and VLb are light chain variable regions; and
VHa and VLa can form the first antigen-binding domain that specifically binds to RANKL, and VHb and VLb can form the second antigen-binding domain that specifically binds to NGF; linker 1 and linker 2 are peptide linkers identical or different in sequence; wherein,
   i) VHa comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
      VLa comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6;
      or
   ii) VHa comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 15; and
      VLa comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
   iii) VHa comprises: a HCDR1 comprising the amino acid sequence of SEQ ID NO: 13; a HCDR2 comprising the amino acid sequence of SEQ ID NO: 8; and a HCDR3 comprising the amino acid sequence of SEQ ID NO: 3; and
      VLa comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; and
      VHb comprises: a HCDR1 comprising the amino acid sequence of SEQ ID NO: 58; a HCDR2 comprising the amino acid sequence of SEQ ID NO: 59; and a HCDR3 comprising the amino acid sequence of SEQ ID NO: 60; and
      VLb comprises: a LCDR1 comprising the amino acid sequence of SEQ ID NO: 61; a LCDR2 comprising the amino acid sequence of SEQ ID NO: 62; and a LCDR3 comprising the amino acid sequence of SEQ ID NO: 63.

In some embodiments, the aforementioned antigen-binding molecule comprises a first chain of the structure of formula (I) and a second chain of the structure of formula (II),

formula (I): VHa-linker 1-VHb-CH1-one subunit of Fc region;

formula (II): VLa-linker 2-VLb-CL;

wherein: VHa and VHb are heavy chain variable regions, and VLa and VLb are light chain variable regions; and
VHa and VLa can form the first antigen-binding domain that specifically binds to RANKL, and VHb and VLb can form the second antigen-binding domain that specifically binds to NGF; linker 1 and linker 2 are peptide linkers identical or different in sequence; wherein,
VHa comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
VLa comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6;
   and
VHb comprises: a HCDR1 comprising the amino acid sequence of SEQ ID NO: 58; a HCDR2 comprising the amino acid sequence of SEQ ID NO: 59; and a HCDR3 comprising the amino acid sequence of SEQ ID NO: 60; and
VLb comprises: a LCDR1 comprising the amino acid sequence of SEQ ID NO: 61; a LCDR2 comprising the amino acid sequence of SEQ ID NO: 62; and a LCDR3 comprising the amino acid sequence of SEQ ID NO: 63.

In some embodiments, the aforementioned antigen-binding molecule comprises a first chain of the structure of formula (I) and a second chain of the structure of formula (II),

formula (I): VHa-linker 1-VHb-CH1-one subunit of Fc region;

formula (II): VLa-linker 2-VLb-CL;

wherein: linker 1 and linker 2 are peptide linkers identical or different in sequence; VHa comprises the amino acid sequence of SEQ ID NO: 36, 37 or 38, and VLa comprises the amino acid sequence of SEQ ID NO: 40; and
VHb comprises the amino acid sequence of SEQ ID NO: 64, and VLb comprises the amino acid sequence of SEQ ID NO: 65.

In some embodiments, the aforementioned antigen-binding molecule comprises a first chain of the structure of formula (I) and a second chain of the structure of formula (II),

formula (I): VHa-linker 1-VHb-CH1-one subunit of Fc region;

formula (II): VLa-linker 2-VLb-CL;

wherein: linker 1 and linker 2 are peptide linkers identical or different in sequence;
VHa comprises the amino acid sequence of SEQ ID NO: 37, and VLa comprises the amino acid sequence of SEQ ID NO: 40; and
VHb comprises the amino acid sequence of SEQ ID NO: 64, and VLb comprises the amino acid sequence of SEQ ID NO: 65.

In some embodiments, in the aforementioned antigen-binding molecule, the linker 1 or linker 2 comprises the amino acid sequence set forth in any one of SEQ ID NOs: 70-97.

In some embodiments, in the aforementioned antigen-binding molecule, the linker 1 comprises the amino acid sequence of SEQ ID NO: 70, and/or the linker 2 comprises the amino acid sequence of SEQ ID NO: 85.

In some embodiments, in the aforementioned antigen-binding molecule, the CH1-Fc region comprises the sequence of SEQ ID NO: 51 or 66.

In some embodiments, the aforementioned antigen-binding molecule comprises:
a first chain, comprising a sequence having at least 85%, 86%, 87%, 88%, 89%, 90%, 91%, 92%, 93%, 94%, 95%, 96%, 97%, 98% or 99% sequence identity to SEQ ID NO: 98, 100 or 101; and/or
a second chain, comprising a sequence having at least 85%, 86%, 87%, 88%, 89%, 90%, 91%, 92%, 93%, 94%, 95%, 96%, 97%, 98% or 99% sequence identity to SEQ ID NO: 99.

In some embodiments, the aforementioned antigen-binding molecule comprises at least two chains:
a first chain, comprising the amino acid sequence of SEQ ID NO: 98, 100 or 101; and a second chain, comprising the amino acid sequence of SEQ ID NO: 99.

In some embodiments, the aforementioned antigen-binding molecule comprises at least two chains:
a first chain, comprising the amino acid sequence of SEQ ID NO: 98; and a second chain, comprising the amino acid sequence of SEQ ID NO: 99.

In some embodiments, the aforementioned antigen-binding molecule comprises two first chains identical in sequence and two second chains identical in sequence.

In some embodiments, the aforementioned antigen-binding molecule comprises two first chains identical in sequence and two second chains identical in sequence, wherein: the first chains comprise the amino acid sequence of SEQ ID NO: 98; and the second chains comprise the amino acid sequence of SEQ ID NO: 99.

In some embodiments, the aforementioned antigen-binding molecule has a DVD-IgG structure comprising a first chain set forth in SEQ ID NO: 98 and a second chain set forth in SEQ ID NO: 99.

In one aspect, the present disclosure also provides an antigen-binding molecule comprising a first antigen-binding domain that specifically binds to RANKL and a second antigen-binding domain that specifically binds to NGF, and the antigen-binding molecule competes with the antigen-binding molecule according to any one of the above for binding to human RANKL and/or human NGF.

In some embodiments, the aforementioned antigen-binding molecule has at least one of the following properties:
i) the affinity with which the first antigen-binding domain that specifically binds to RANKL binds to RANKL is higher than the affinity with which the second antigen-binding domain that specifically binds to NGF binds to NGF;
ii) the EC50 value with which the first antigen-binding domain that specifically binds to RANKL binds to human RANKL is less than the EC50 value with which the second antigen-binding domain that specifically binds to NGF binds to human NGF; and
iii) the IC50 value with which the first antigen-binding domain that specifically binds to RANKL blocks binding of RANKL to RANK is less than the IC50 value with which the second antigen-binding domain that specifically binds to NGF blocks binding of NGF to TrKA.

In some embodiments, the aforementioned antigen-binding molecule has at least one of the following properties:
i) the affinity with which the first antigen-binding domain that specifically binds to RANKL binds to RANKL is higher than the affinity with which the second antigen-binding domain that specifically binds to NGF binds to NGF, as determined by Biacore;
ii) the EC50 value with which the first antigen-binding domain that specifically binds to RANKL binds to human RANKL is less than the EC50 value with which the second antigen-binding domain that specifically binds to NGF binds to human NGF, as determined by ELISA; and
iii) the IC50 value with which the first antigen-binding domain that specifically binds to RANKL blocks binding of RANKL to RANK is less than the IC50 value with which the second antigen-binding domain that specifically binds to NGF blocks binding of NGF to TrKA, as determined by ELISA.

In some embodiments, the aforementioned antigen-binding molecule has at least one of the following properties:
i) the affinity with which the first antigen-binding domain that specifically binds to RANKL binds to RANKL is at least 2 times, at least 3 times, at least 4 times, or at least 5 times higher than the affinity with which the second antigen-binding domain that specifically binds to NGF binds to NGF, as determined by Biacore;
ii) the EC50 value with which the first antigen-binding domain that specifically binds to RANKL binds to human RANKL is at least 25 times, at least 20 times, at least 15 times, at least 10 times, at least 9 times, at least 8 times, at least 4 times, or at least 2 times lower than the EC50 value with which the second antigen-binding domain that specifically binds to NGF binds to human NGF, as determined by ELISA; and
iii) the IC50 value with which the first antigen-binding domain that specifically binds to RANKL blocks binding of RANKL to RANK is at least 20 times, at least 15 times, at least 10 times, at least 8 times, at least 4 times, or at least 2 times lower than the IC50 value with which the second antigen-binding domain that specifically binds to NGF blocks binding of NGF to TrKA, as determined by ELISA.

In some embodiments, the aforementioned antigen-binding molecule has at least one of the following properties:
i) the first antigen-binding domain that specifically binds to RANKL binds to RANKL with a KD value of less than 5 nM, less than 4 nM, less than 3 nM, or less than 2 nM; and
   the second antigen-binding domain that specifically binds to NGF binds to NGF with a KD value of greater than 10 nM;
ii) the first antigen-binding domain that specifically binds to RANKL binds to human RANKL with an EC50 of less than 0.13 nM, and
   the second antigen-binding domain that specifically binds to NGF binds to human NGF with an EC50 of greater than 3 nM; and
iii) the first antigen-binding domain that specifically binds to RANKL blocks binding of RANKL to RANK with an IC50 of less than 0.5 nM; and
   the second antigen-binding domain that specifically binds to NGF blocks binding of NGF to TrKA with an IC50 of greater than 4 nM.

In one aspect, the present disclosure provides an anti-RANKL antibody with a relatively high affinity.

In some embodiments, the aforementioned anti-RANKL antibody comprises a heavy chain variable region and a light chain variable region, wherein:
i) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
ii) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 15; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
iii) the heavy chain variable region comprises: a HCDR1 comprising the amino acid sequence of SEQ ID NO: 13; a HCDR2 comprising the amino acid sequence of SEQ ID NO: 8; and a HCDR3 comprising the amino acid sequence of SEQ ID NO: 3; and
   the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6.

In some embodiments, the aforementioned anti-RANKL antibody comprises a light chain variable region and a heavy chain variable region, wherein:
i) the heavy chain variable region comprises a HCDR1, a HCDR2 and a HCDR3 set forth in SEQ ID NO: 7, SEQ ID NO: 8 and SEQ ID NO: 14, respectively; and
   the light chain variable region comprises a LCDR1, a LCDR2 and a LCDR3 set forth in SEQ ID NO: 4, SEQ ID NO: 5 and SEQ ID NO: 6, respectively; or
ii) the heavy chain variable region comprises a HCDR1, a HCDR2 and a HCDR3 set forth in SEQ ID NO: 7, SEQ ID NO: 8 and SEQ ID NO: 15, respectively; and
   the light chain variable region comprises a LCDR1, a LCDR2 and a LCDR3 set forth in SEQ ID NO: 4, SEQ ID NO: 5 and SEQ ID NO: 6, respectively; or
iii) the heavy chain variable region comprises a HCDR1, a HCDR2 and a HCDR3 set forth in SEQ ID NO: 13, SEQ ID NO: 8 and SEQ ID NO: 3, respectively; and
   the light chain variable region comprises a LCDR1, a LCDR2 and a LCDR3 set forth in SEQ ID NO: 4, SEQ ID NO: 5 and SEQ ID NO: 6, respectively.

In some embodiments, the aforementioned anti-RANKL antibody comprises a light chain variable region and a heavy chain variable region, wherein the heavy chain variable region comprises an amino acid sequence having at least 90%, 90%, 91%, 92%, 93%, 94%, 95%, 96%, 97%, 98% or 99% sequence identity to SEQ ID NO: 36, 37 or 38; and/or
the light chain variable region comprises an amino acid sequence having at least 90%, 90%, 91%, 92%, 93%, 94%, 95%, 96%, 97%, 98% or 99% sequence identity to SEQ ID NO: 40.

In some embodiments, the aforementioned anti-RANKL antibody comprises a light chain variable region and a heavy chain variable region, wherein the heavy chain variable region comprises an amino acid sequence having at least 90%, 90%, 91%, 92%, 93%, 94%, 95%, 96%, 97%, 98% or 99% sequence identity to SEQ ID NO: 37; and/or
the light chain variable region comprises an amino acid sequence having at least 90%, 90%, 91%, 92%, 93%, 94%, 95%, 96%, 97%, 98% or 99% sequence identity to SEQ ID NO: 40.

In some embodiments, the aforementioned anti-RANKL antibody comprises a light chain variable region and a heavy chain variable region, wherein the heavy chain variable region comprises the amino acid sequence of SEQ ID NO: 36, 37 or 38; and/or the light chain variable region comprises the amino acid sequence of SEQ ID NO: 40.

In some embodiments, the aforementioned anti-RANKL antibody comprises a light chain variable region and a heavy chain variable region, wherein the sequence of the heavy chain variable region is set forth in SEQ ID NO: 36, 37 or 38; and/or the sequence of the light chain variable region is set forth in SEQ ID NO: 40.

In some embodiments, the aforementioned anti-RANKL antibody comprises a heavy chain variable region and a light chain variable region, wherein the heavy chain variable region comprises the amino acid sequence of SEQ ID NO: 37; and/or the light chain variable region comprises the amino acid sequence of SEQ ID NO: 40.

In some embodiments, the aforementioned anti-RANKL antibody comprises a heavy chain variable region and a light chain variable region, wherein the heavy chain variable region comprises the amino acid sequence of SEQ ID NO: 36; and/or the light chain variable region comprises the amino acid sequence of SEQ ID NO: 40.

In some embodiments, the aforementioned anti-RANKL antibody comprises a heavy chain variable region and a light chain variable region, wherein the heavy chain variable region comprises the amino acid sequence of SEQ ID NO: 38; and/or the light chain variable region comprises the amino acid sequence of SEQ ID NO: 40.

In some embodiments, the aforementioned anti-RANKL antibody comprises a heavy chain variable region and a light chain variable region, wherein the sequence of the heavy chain variable region is set forth in SEQ ID NO: 37; and/or the sequence of the light chain variable region is set forth in SEQ ID NO: 40.

In some embodiments, the aforementioned anti-RANKL antibody comprises a heavy chain constant region and a light chain constant region.

In some embodiments, the aforementioned anti-RANKL antibody comprises an IgG1, IgG2, IgG3 or IgG4 heavy chain constant region and a λ chain or κ chain light chain constant region.

In some embodiments, in the aforementioned anti-RANKL antibody, the heavy chain constant region comprises the amino acid sequence of SEQ ID NO: 51, and/or the light chain constant region comprises the amino acid sequence of SEQ ID NO: 52.

In some embodiments, the aforementioned anti-RANKL antibody comprises a heavy chain and a light chain, wherein:
the heavy chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 53, 55 or 56; and/or the light chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 54.

In some embodiments, the aforementioned anti-RANKL antibody comprises a heavy chain and a light chain, wherein:
the heavy chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 53; and/or the light chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 54.

In some embodiments, the aforementioned anti-RANKL antibody comprises a heavy chain and a light chain, wherein:
the heavy chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 55; and/or the light chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 54.

In some embodiments, the aforementioned anti-RANKL antibody comprises a heavy chain and a light chain, wherein:
the heavy chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 56; and/or the light chain comprises an amino acid sequence having at least 85%, 86%, 87%, 88%, 89%, 90%, 91%, 92%, 93%, 94%, 95%, 96%, 97%, 98% or 99% sequence identity to SEQ ID NO: 54.

In some embodiments, the aforementioned anti-RANKL antibody comprises a heavy chain and a light chain, wherein:
the heavy chain comprises the amino acid sequence of SEQ ID NO: 53, and/or the light chain comprises the amino acid sequence of SEQ ID NO: 54; or
the heavy chain comprises the amino acid sequence of SEQ ID NO: 55, and/or the light chain comprises the amino acid sequence of SEQ ID NO: 54; or
the heavy chain comprises the amino acid sequence of SEQ ID NO: 56, and/or the light chain comprises the amino acid sequence of SEQ ID NO: 54.

In some embodiments, the aforementioned anti-RANKL antibody comprises a heavy chain and a light chain, wherein:
the sequence of the heavy chain is set forth in SEQ ID NO: 53, and/or the sequence of the light chain is set forth in SEQ ID NO: 54; or
the sequence of the heavy chain is set forth in SEQ ID NO: 55, and/or the sequence of the light chain is set forth in SEQ ID NO: 54; or
the sequence of the heavy chain is set forth in SEQ ID NO: 56, and/or the sequence of the light chain is set forth in SEQ ID NO: 54.

In some embodiments, the aforementioned anti-RANKL antibody is an antibody fragment; preferably, the antibody fragment is selected from the group consisting of Fab, Fab', F(ab')2, Fd, Fv, scFv, dsFv and dAb.

In some embodiments, the aforementioned anti-RANKL antibody has the following properties:
the antibody binds to human RANKL with a KD value of less than 5 nM; and/or
the antibody blocks binding of RANKL to RANK with an IC50 value of less than 0.5 nM.

In one aspect, the present disclosure also provides a bispecific antibody comprising the anti-RANKL antibody according to any one of the above.

In one aspect, the present disclosure provides an isolated nucleic acid encoding the antigen-binding molecule according to any one of the above, or the anti-RANKL antibody according to any one of the above, or the bispecific antibody according to the above.

In one aspect, the present disclosure provides a vector comprising the nucleic acid according to the above.

In one aspect, the present disclosure provides a host cell comprising the vector according to the above.

The host cell of the present application cannot develop into a complete animal or plant individual.

In one aspect, the present disclosure provides a method for preparing an antigen-binding molecule or an anti-RANKL antibody or a bispecific antibody, comprising culturing the host cell according to the above under conditions suitable for expression of the antigen-binding molecule or the anti-RANKL antibody or the bispecific antibody.

In one aspect, the present disclosure provides a pharmaceutical composition comprising: a prophylactically or therapeutically effective amount of the antigen-binding molecule according to any one of the above, or the anti-RANKL antibody according to any one of the above, or the bispecific antibody according to the above, and one or more pharmaceutically acceptable carriers, diluents, buffers or excipients.

In one aspect, the present disclosure provides a method for treating or preventing a related disease, the method comprising administering to a subject a prophylactically or therapeutically effective amount of the antigen-binding molecule according to the above, or the anti-RANKL antibody according to any one of the above, or the bispecific antibody according to the above, or the pharmaceutical composition according to the above.

In some embodiments, the disease is pain, joint stiffness or bone loss.

In some embodiments, the pain is selected from the group consisting of osteoarticular pain, rheumatoid arthritis pain, gout, bone cancer pain, post-surgical pain, painful bladder syndrome, chronic prostatitis, chronic pelvic pain, lower back pain, pain associated with bone disease, and pain associated with peripheral nerves.

In some embodiments, the bone loss is associated with at least one condition selected from the group consisting of osteoporosis, Paget's disease, osteomyelitis, hypercalcemia, osteopenia, osteonecrosis, inflammation, autoimmune disease, rheumatoid arthritis, periodontal bone resorption, osteolytic metastasis, and cancer. In some embodiments, the cancer is selected from the group consisting of breast cancer, prostate cancer, thyroid cancer, kidney cancer, lung cancer, esophageal cancer, rectal cancer, bladder cancer, cervical cancer, ovarian cancer, liver cancer, gastrointestinal cancer, melanoma, multiple myeloma, osteosarcoma, lymphoma, non-small cell lung cancer, bone tumor, and Hodgkin's disease.

In some embodiments, the aforementioned disease is an NGF- or RANKL-associated disease.

In some embodiments, the aforementioned disease is a disease that expresses NGF or RANKL.

In one aspect, the present disclosure provides a method for treating or preventing an NGF- or RANKL-associated disease, the method comprising administering to a subject a prophylactically or therapeutically effective amount of the antigen-binding molecule according to any one of the above, or the anti-RANKL antibody according to any one of the above, or the bispecific antibody according to the above, or the pharmaceutical composition according to the above.

In some embodiments, the NGF- or RANKL-associated disease is pain, joint stiffness or bone loss.

In one aspect, the present disclosure also provides a use of the antigen-binding molecule according to any one of the above, or the anti-RANKL antibody according to any one of the above, or the bispecific antibody according to the above, or the pharmaceutical composition according to the above, in the preparation of a medicament for preventing or treating a disease or disorder. In some embodiments, the disease is pain, joint stiffness or bone loss.

In some embodiments, the pain is selected from the group consisting of osteoarticular pain, rheumatoid arthritis pain, gout, bone cancer pain, post-surgical pain, painful bladder syndrome, chronic prostatitis, chronic pelvic pain, lower back pain, pain associated with bone disease, and pain associated with peripheral nerves. In some embodiments, the bone loss is associated with at least one condition selected from the group consisting of osteoporosis, Paget's disease, osteomyelitis, hypercalcemia, osteopenia, osteonecrosis, inflammation, autoimmune disease, rheumatoid arthritis, periodontal bone resorption, osteolytic metastasis, and cancer. In some embodiments, the cancer is selected from the group consisting of breast cancer, prostate cancer, thyroid cancer, kidney cancer, lung cancer, esophageal cancer, rectal cancer, bladder cancer, cervical cancer, ovarian cancer, liver cancer, gastrointestinal cancer, melanoma, multiple myeloma, osteosarcoma, lymphoma, non-small cell lung cancer, bone tumor, and Hodgkin's disease.

In one aspect, the antigen-binding molecule according to any one of the above, or the anti-RANKL antibody according to any one of the above, or the bispecific antibody according to the above, or the pharmaceutical composition according to the above, of the present disclosure, can be used as a medicament. In some embodiments, provided is use as a medicament for treating pain, joint stiffness or bone loss. In some embodiments, provided is use as a medicament for treating osteoarticular pain, rheumatoid arthritis pain, gout, bone cancer pain, post-surgical pain, painful bladder syndrome, chronic prostatitis, chronic pelvic pain, lower back pain, pain associated with bone disease, and pain associated with peripheral nerves. In some embodiments, provided is use as a medicament for treating bone loss caused by osteoporosis, Paget's disease, osteomyelitis, hypercalcemia, osteopenia, osteonecrosis, inflammation, autoimmune disease, rheumatoid arthritis, periodontal bone resorption, osteolytic metastasis, and cancer.

In one aspect, the present disclosure provides use of the antigen-binding molecule according to any one of the above, or the anti-RANKL antibody according to any one of the above, or the bispecific antibody according to the above, or the pharmaceutical composition according to the above, in the preparation of a medicament for preventing or treating an NGF- or RANKL-associated disease. In some embodiments, the NGF- or RANKL-associated disease is pain, joint stiffness or bone loss.

In one aspect, the antigen-binding molecule according to any one of the above, or the anti-RANKL antibody according to any one of the above, or the bispecific antibody according to the above, or the pharmaceutical composition according to the above, provided by the present disclosure, can be used as a medicament for preventing or treating an NGF- or RANKL-associated disease. In some embodiments, the NGF- or RANKL-associated disease is pain, joint stiffness or bone loss.

In one aspect, the present disclosure provides a method for preventing or treating pain or inhibiting bone loss, the method comprising administering to the individual an effective amount of an NGF antagonist and a RANKL antagonist; the NGF antagonist and the RANKL antagonist are administered simultaneously or sequentially.

In one aspect, the present disclosure provides use of an NGF antagonist and a RANKL antagonist in the preparation of a medicament for preventing or treating pain or inhibiting bone loss.

In one aspect, the present disclosure provides use of an NGF antagonist in combination with a RANKL antagonist, for treating pain or inhibiting bone loss; the NGF antagonist and the RANKL antagonist are administered simultaneously or sequentially.

In some embodiments, the NGF antagonist and the RANKL antagonist are in the same container or different containers.

In one aspect, the present disclosure provides an NGF antagonist for use in preventing or treating pain or inhibiting bone loss, wherein the NGF antagonist is used in combination with a RANKL antagonist, and the NGF antagonist and the RANKL antagonist are not in the same container.

In one aspect, the present disclosure provides a RANKL antagonist for use in preventing or treating pain or inhibiting bone loss, wherein the RANKL antagonist is used in combination with an NGF antagonist, and the NGF antagonist and the RANKL antagonist are not in the same container.

In one aspect, the present disclosure provides a composition comprising an NGF antagonist and a RANKL antagonist, for use in preventing or treating pain or inhibiting bone loss.

In some embodiments, the NGF antagonist is an anti-NGF antibody.

In some embodiments, the RANKL antagonist is an anti-RANKL antibody.

In some embodiments, the NGF antagonist and the RANKL antagonist are prepared as a mixture.

In some other embodiments, the NGF antagonist and the RANKL antagonist are not in the form of a mixture, and the NGF antagonist and the RANKL antagonist are contained in different containers, or packaged in different kits.

It should be understood that the NGF antagonist and the RANKL antagonist are allowed to be prepared separately in practical clinical applications, but are clinically administered in combination. In instructions for use of the NGF antagonist/RANKL antagonist, if it is indicated that the NGF antagonist is administered in combination with the RANKL antagonist, it falls within "use of the NGF antagonist and the RANKL antagonist in the preparation of a medicament for preventing or treating disease".

In some embodiments, the NGF antagonist and the RANKL antagonist are administered simultaneously or sequentially. It should be understood that "simultaneous" is not to be interpreted as one and the same point in time. Where the NGF antagonist and the RANKL antagonist are administered at an interval of no more than 30 min (inclusive), it can be considered as "simultaneous administration". Where the two are administered at an interval of more than 30 min, it can be considered as "sequential administration". The upper limit of the interval between their administrations is defined as the point where they can still produce a synergistic effect.

In some embodiments, the anti-NGF antibody is tanezumab, and/or the anti-RANKL antibody is AMR2.

In some embodiments, the anti-NGF antibody is tanezumab, and/or the anti-RANKL antibody is denosumab.

The antigen-binding molecule provided by the present disclosure has the following characteristics: good therapeutic activity, safety, pharmacokinetic properties and druggability (e.g., stability).

### BRIEF DESCRIPTION OF THE DRAWINGS

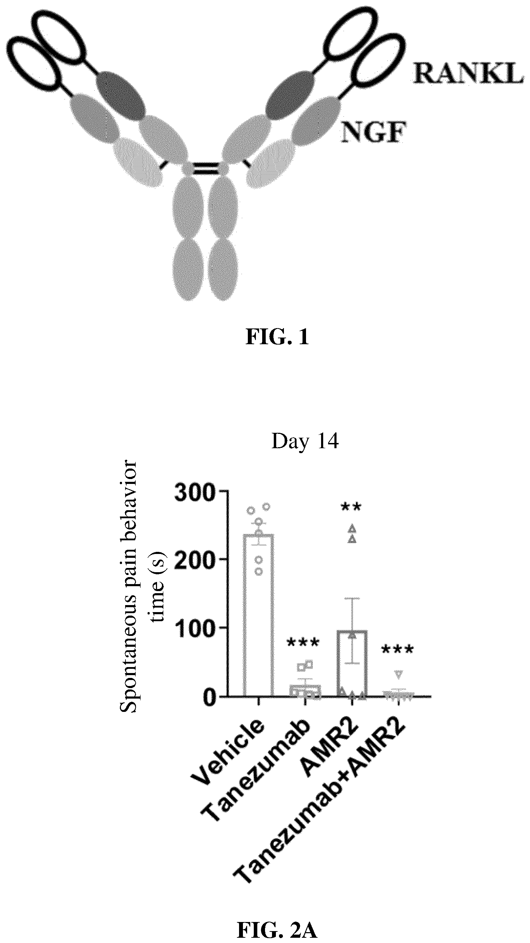

FIG. 1: a diagram of the DVD-IgG structure.

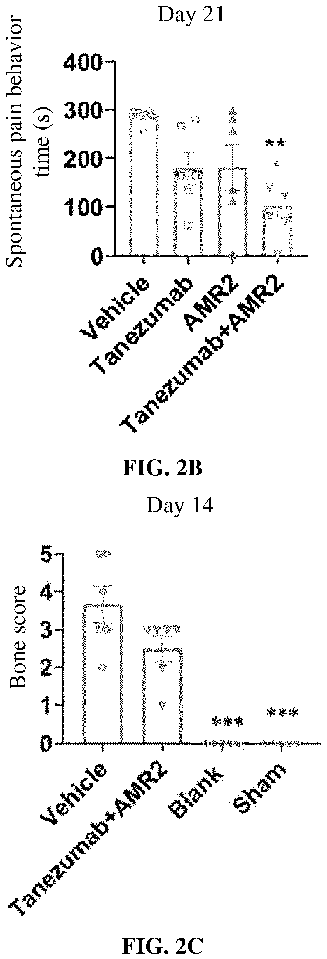

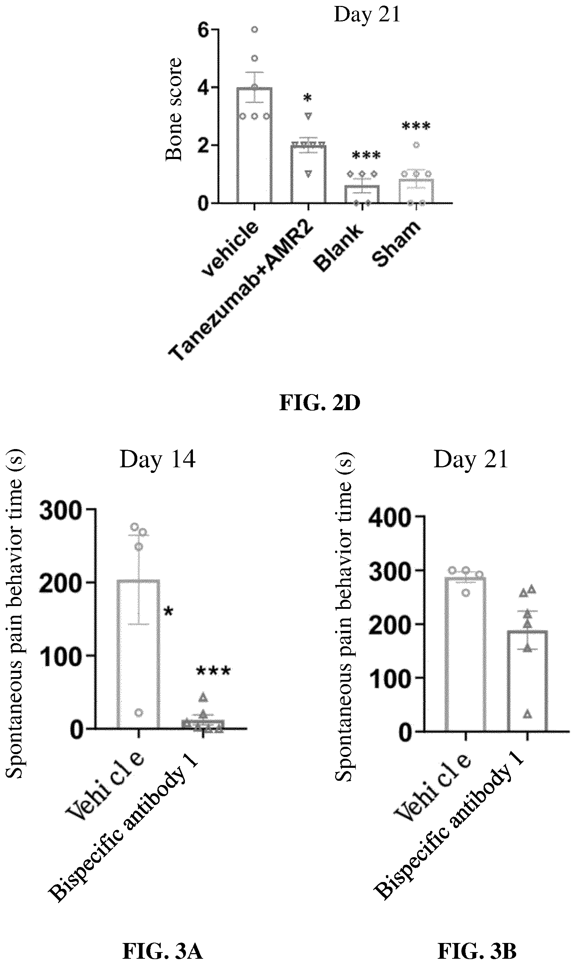

FIG. 2A to FIG. 2D: FIG. 2A shows statistical results of pain behaviors in mice on day 14 of combination therapy; FIG. 2B shows statistical results of pain behaviors in mice on day 21 of combination therapy; FIG. 2C shows bone injury scores of mice on day 14 of combination therapy; FIG. 2D shows bone injury scores of mice on day 21 of combination therapy; wherein vs vehicle; **** represents vs vehicle, P < 0.0001; *** represents vs vehicle, P < 0.001; * represents vs vehicle, P < 0.05. Blank; Sham.
FIG. 3A to FIG. 3B: FIG. 3A shows statistical results of pain behaviors in mice on day 14 of using bispecific antibody 1; FIG. 3B shows statistical results of pain behaviors in mice on day 21 of using bispecific antibody 1; wherein vs vehicle **** represents vs vehicle, P < 0.0001; *** represents vs vehicle, P < 0.001; * represents vs vehicle, P < 0.05.

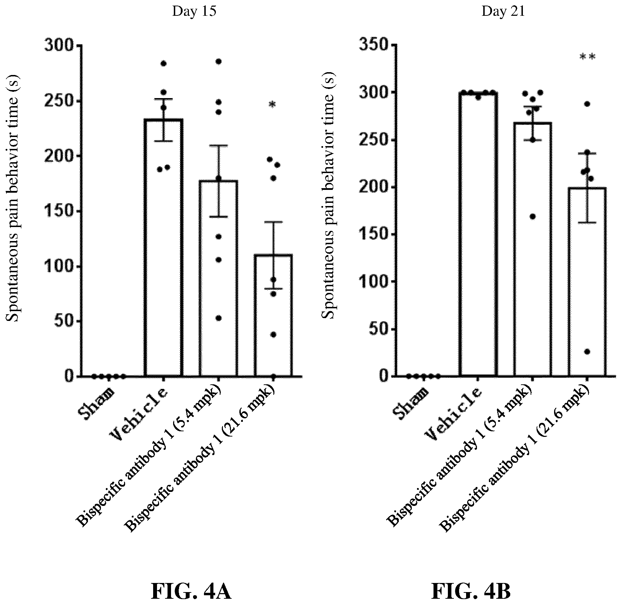

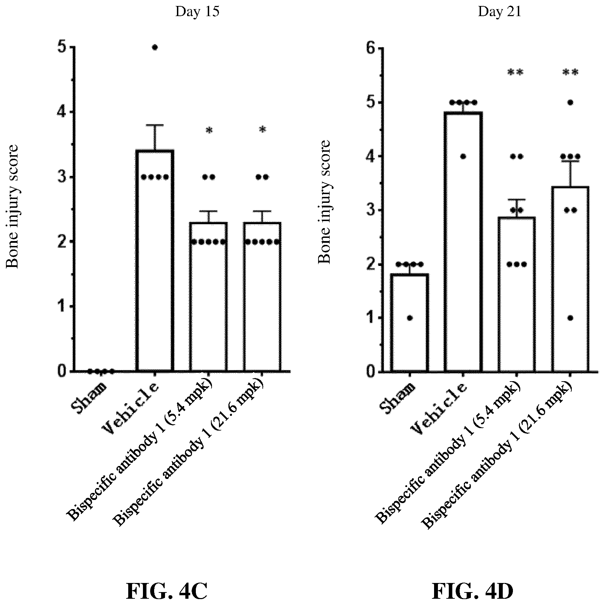

FIG. 4A to FIG. 4D: FIG. 4A shows statistical results of pain behaviors in mice on day 15 of using bispecific antibody 1; FIG. 4B shows statistical results of pain behaviors in mice on day 21 of using bispecific antibody 1; FIG. 4C shows bone injury scores of mice on day 15 of using bispecific antibody 1; FIG. 4D shows bone injury scores of mice on day 21 of using bispecific antibody 1; wherein vs vehicle **** represents vs vehicle, P < 0.0001; *** represents vs vehicle, P < 0.001; * represents vs vehicle, P < 0.05. Sham, vehicle.

### DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

As used in the description and the claims, the singular forms "a", "an" and "the" include plural reference unless the context clearly dictates otherwise.

Unless the context clearly requires otherwise, throughout the description and the claims, the words "comprise", "have", "include", and the like are to be construed in an inclusive sense as opposed to an exclusive or exhaustive sense; that is to say, in the sense of "including, but not limited to". Unless otherwise indicated, "comprise" includes "consist of'.

The three-letter and single-letter codes used in the present disclosure for amino acids are as described in J. biol. chem, 243, p3558 (1968).

The term "and/or", e.g., "X and/or Y" should be understood to mean "X and Y" or "X or Y" and should be used to provide explicit support for both meanings or either meaning.

The term "antigen-binding molecule" is used in the broadest sense and encompasses a variety of molecules that specifically bind to an antigen or epitope thereof, including but not limited to antibodies, other polypeptides having antigen-binding activity, and antibody fusion proteins in which the two are fused. Illustratively, the antigen-binding molecules herein are bispecific antigen-binding molecules (e.g., bispecific antibodies) that can comprise two identical (or different) first chains and two identical (or different) second chains; or a first chain, a second chain, a third chain and a fourth chain that are different from each other. Illustratively, the chains are polypeptide chains. Illustratively, the first polypeptide chain or the third polypeptide chain may be a heavy chain of an antibody or a polypeptide comprising an Fc region, and the second polypeptide chain or the fourth polypeptide chain may be a light chain of an antibody or an engineered antibody light chain. The term "bispecific antigen-binding molecule" refers to an antigen-binding molecule capable of specifically binding to two different antigens or at least two different epitopes of the same antigen.

The term "RANK antagonist" refers to an agent, which is a RANKL binder that statistically significantly neutralizes RANKL and/or reduces or inhibits the binding of RANKL to its receptor RANK, thereby antagonizing the RANK-RANKL interaction and its downstream signaling pathways.

The antagonistic function of the RANK antagonist is particularly characterized by diminishing, inhibiting or preventing a response to a receptor (RANK) activated by an agonist (RANKL). Antagonists, particularly competitive antagonists, can reversibly bind to RANKL at the same binding site or, as an endogenous receptor, interfere with the binding site (active site) without having to activate the receptor.

The agent can be any suitable binder or ligand, e.g., selected from the group consisting of small organic or inorganic molecules, carbohydrates, biological macromolecules, peptides, proteins (herein also referred to as polypeptides), peptide analogs, peptidomimetics, antibodies (including antigen-binding fragments of antibodies), nucleic acids, nucleic acid analogs, and a combination of any of the foregoing. In some embodiments, the RANKL antagonist is an immunotherapeutic agent. A specific example of the agent is selected from the group consisting of an antibody, a receptor or osteoprotegerin (which is inactive or rendered inactive in order to avoid the binding of the agonist RANKL to its receptor RANK), a receptor-fusion protein, e.g., a RANK-Fc fusion protein, a peptide, an aptamer or a small molecule. Exemplary RANKL antagonists include denosumab, recombinant RANK-Fc (Schmiedel et al. 2013, Cancer Res. 73(2):683-94), RANKL-nanobodies (WO2008142164A2), or RANKL-binding peptides (WO2012163887A1).

By "statistically significantly reduces or inhibits" the RANK-RANKL interaction and its downstream signaling pathways is meant that the antagonist exhibits any one selected from the group consisting of the following or a combination thereof:
(i) the antagonist inhibits the binding of RANKL to RANK by more than 50%, preferably by more than 60%, 70%, 80%, 90% or 95%, or inhibits such binding completely;
(ii) the antagonist functionally inhibits RANKL-induced pathways, in particular the signaling following RANK stimulation, e.g., the activity of MAPK (mitogen-activated protein kinase) or SRC kinases, or NF-κB signals involved in metastasis formation; or
(iii) at certain significant levels (e.g., without limitation, p is set to 0.5, 0.1, 0.05, 0.01, 0.005, 0.0001, 0.0005, 0.0001, or even lower), there is a significant difference in the RANK-RANKL interaction level (or the level of activity of a member in its downstream signaling pathway) between a group in which the antagonist is present and a group in which the antagonist is absent.

Such functional inhibition is, for example, inhibition of metastasis formation to a degree of greater than about 50%, 60%, 70%, 80%, 90% or 95%, or complete inhibition.

The term "denosumab" includes biological analogs of denosumab. As used herein, "biological analog" (of an approved reference product/biological drug, such as a protein therapeutic agent or antibody) refers to a biological product that is similar to the reference product based on data derived from: (a) analytical studies that show that the biological product is highly similar to the reference product, but there are minor differences in clinically inactive components; (b) animal studies (including toxicity assessment); and/or (c) one or more clinical studies (including the assessment of immunogenicity and pharmacokinetics or pharmacodynamics) that are sufficient to demonstrate safety, purity and potency in one or more appropriate conditions of use for which the reference product is licensed and intended to be used and for which licensure is sought for the biological product.

"Anti-RANKL antibody" refers to an antibody that is capable of binding to RANKL or an epitope thereof and inhibiting the biological activity of RANKL and/or inhibiting downstream pathways of RANKL.

The term "NGF" or nerve growth factor refers to a nerve growth factor and variants thereof that retain at least part of the biological activity of NGF. As used herein, NGF includes the wild-type sequence NGF or naturally occurring variants thereof of all mammalian species, including human, canine, feline, equine or bovine.

"NGF receptor" refers to a polypeptide that binds to NGF or is activated by NGF. NGF receptors include the TrkA receptor and the p75 receptor of any mammalian species, including but not limited to, human, canine, feline, equine, primate or bovine.

"NGF antagonist" refers to any compound that neutralizes NGF signaling and biological activity. For example, an NGF antagonist can inhibit the binding of NGF to its ligand, such as the TrkA or p75 receptor that binds to the cell membrane.

In some embodiments, the NGF antagonist includes anti-NGF antibodies, fragments of anti-NGF antibodies, or NGF-binding proteins, or NGF-binding polypeptides. In certain embodiments, the NGF antagonist is designed for the particular species to be treated. For example, when the species is human, a human or humanized NGF antagonist is used, such as RN624 (tanezumab), JNJ-42160443, REGN475, PG110, alpha-D11, AMG-403 (see, for example, Mantyh et al., Anaesthesiology 115:189-204 (2011)), the 911 antibody described in Hongo et al., Hybhdoma: 19:215-27 (2000), or the aD11 antibody described in Ruberti et al., Cell. Molec. Neurobiol. 13:559-68 (1993), or any of those described in WO 2012153121, WO 2012153122, WO 2012153123 and WO 2012153126.

In some embodiments, the NGF antagonist is an NGF-binding protein or polypeptide, e.g., a polypeptide comprising TrkA or p75, or a fragment of TrkA or p75 that can bind to NGF. In a specific embodiment, the NGF antagonist is an NGF-binding polypeptide comprising TrkA or p75 (or an NGF-binding fragment thereof) and the Fc of an immunoglobulin. In some embodiments, the NGF antagonist is a TrkA-IgG fusion protein that can bind to NGF, for example, those described in Shelton et al., J. Neurosci. 15:477-91 (1995). In some embodiments, the NGF antagonist is a p75 fusion protein that binds to NGF, such as p75-Fc developed by Levicept (Kent, England) for treating chronic pain. In some embodiments, the NGF antagonist is a small-molecule compound that can interfere with the binding of NGF to TrkA, such as ALE0540 and PD90780, see Hefti et al., TRENDS in Pharmacol. Sci. 27:85-91 (2006); or MNAC13, see Mantyh et al., Anaesthesiology 115:189-204 (2011). In some embodiments, the NGF antagonist is a small-molecule compound that inhibits TrkA.

"Biological activity" of NGF generally refers to the ability to bind to NGF receptors and/or activate NGF receptor signaling pathways. Biological activity includes, but is not limited to, one or more of the following: the ability to bind to an NGF receptor (such as p75 and/or trkA); the ability to promote TrkA receptor dimerization and/or autophosphorylation; the ability to activate an NGF receptor signaling pathway; the ability to promote cell differentiation, proliferation, survival, growth and other changes in cell physiology, including change in neuronal (including peripheral and central neurons) morphology, synaptogenesis, synaptic function, neurotransmitter and/or neuropeptide release and regeneration following damage; the ability to promote survival of trigeminal neurons; and the ability to modulate pain, including post-surgical pain.

"Anti-NGF antibody" refers to an antibody that is capable of binding to NGF and inhibiting the biological activity of NGF and/or downstream pathways modulated by NGF signaling. Anti-NGF antibodies include antibodies that block, antagonize, suppress or reduce NGF biological activity, including downstream pathways modulated by NGF signaling, such as receptor binding and/or elicitation of a response to NGF. In some specific embodiments, the anti-NGF antibody binds to NGF and prevents NGF dimerization and/or binding to NGF receptors (e.g., p75 and/or TrkA). In other specific embodiments, the anti-NGF antibody binds to NGF and prevents TrkA receptor dimerization and/or trkA autophosphorylation.

The term "tanezumab" includes biological analogs of tanezumab.

The term "amino acid" refers to naturally occurring and synthetic amino acids, as well as amino acid analogs or amino acid mimics that function in a similar manner to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton but retain a substantially identical chemical structure to naturally occurring amino acids. Amino acid mimics refer to chemical compounds that have a structure different from the general chemical structure of amino acids but function in a similar manner to naturally occurring amino acids.

The term "amino acid mutation" includes amino acid substitutions, deletions, insertions and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., a replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W. The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), so long as they exhibit the desired antigen-binding activity. "Native antibody" refers to a naturally-occurring immunoglobulin molecule. For example, a native IgG antibody is a heterotetrameric glycoprotein of about 150,000 Daltons composed of two light chains and two heavy chains linked by a disulfide bond. From N-terminus to C-terminus, each heavy chain has one variable region (VH, also known as variable heavy domain or heavy chain variable region), followed by three constant domains (CH1, CH2, and CH3). Similarly, from the N-terminus to the C-terminus, each light chain has one variable region (VL, also known as variable light domain or light chain variable domain), followed by one constant light domain (light chain constant region, CL). The term "full-length antibody", "intact antibody", and "whole antibody" are used herein interchangeably and refer to an antibody having a substantially similar structure to a native antibody structure or having heavy chains comprising an Fc region as defined herein.

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of horizontal symmetry in their structures. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type comprise bispecific antibodies, such as F(ab)2, scFv-Fab and (scFv)2-Fab; IgG-like bispecific antibodies (e.g., having Fc fragments) have relatively large molecular weight, and the Fc fragments facilitate later purification of the antibody and increase their solubility and stability, and the Fc fragments may further bind to the receptor FcRn and increase the serum half-life of the antibody, and typical structural models of bispecific antibodies comprise bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2 and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain, which is involved in antibody-antigen interactions, of a heavy chain or light chain. The heavy chain variable region can be denoted by VH and the light chain variable region by VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3.

Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), and the like. The corresponding relationships among the various numbering schemes are well known to those skilled in the art and are exemplary, as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in the embodiments of the present disclosure.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise indicated, the numbering scheme for the Fc region is the EU index.

The term "affinity" refers to the overall strength of the non-covalent interaction between a single binding site of a molecule (e.g., an antibody) and its binding ligand (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally denoted by the dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the dissociation constant, which is obtained from the ratio of kd to ka (i.e., kd/ka) and expressed as a molar concentration (M). The KD values of antibodies can be determined by methods known in the art, for example, methods for determining antibody KD include measuring surface plasmon resonance using a biosensing system, such as a system, or measuring affinity in solution by solution equilibrium titration (SET) assay. The term "monoclonal antibody" refers to a population of substantially homogeneous antibodies, that is, the amino acid sequences of the antibody molecules comprised in the population are identical, except for a small number of natural mutations that may exist. In contrast, polyclonal antibody preparations generally comprise a plurality of different antibodies having different amino acid sequences in their variable domains, which are generally specific for different epitopes. "Monoclonal" refers to the characteristics of an antibody obtained from a population of substantially homogeneous antibodies, and should not be construed as requiring the production of the antibody by any particular method. In some embodiments, the antibody provided by the present disclosure is a monoclonal antibody.

The term "antigen" refers to a molecule that is capable of being selectively bound by an antigen-binding protein (e.g., an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g. coming in spatial proximity due to the folding of the antigen (i.e. by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

An "antibody that competes for binding with" a reference antibody refers to an antibody that blocks binding of the reference antibody to an antigen by 50% or more, or an antibody whose binding to an antigen is blocked by 50% or more by the reference antibody, in a competition assay.

The term "capable of specifically binding", "specifically binding", or "binding" means that an antigen-binding molecule is capable of binding to a certain antigen or an epitope thereof with higher affinity than to other antigens or epitopes. Generally, an antibody binds to an antigen or epitope thereof with an equilibrium dissociation constant (KD) of about 1×10⁻⁷ M or less (e.g., about 1×10⁻⁸ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE^{®} surface plasmon resonance assay. However, an antibody that specifically binds to an antigen or an epitope thereof may have cross-reactivity to other related antigens, e.g. to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "anti-RANKL antibody" or "antigen binding domain that specifically binds to RANKL" refers to an antibody or domain that is capable of binding to RANKL or an epitope thereof with such sufficient affinity that the anti-RANKL antibody or an antibody containing the domain can be used as a diagnostic and/or therapeutic agent targeting RANKL. In certain embodiments, the antibody or domain that binds to RANKL has a dissociation constant (KD) of < about 1 µM, < about 100 nM, or < about 10 nM, as determined by FACS. In certain embodiments, the anti-RANKL antibody or the antigen-binding domain that specifically binds to RANKL binds to a conserved RANKL epitope of RANKL from different species.

The term "anti-NGF antibody" or "antigen-binding domain that specifically binds to NGF" refers to an antibody that is capable of binding to NGF with such sufficient affinity that an antibody containing the domain can be used as a diagnostic and/or therapeutic agent targeting NGF. In certain embodiments, the anti-NGF antibody binds to a conserved NGF epitope of NGF from different species.

The term "linker" refers to a linker unit that links two polypeptide fragments. Herein, the linkers present in the same molecule may be identical or different. The linkers may be peptide linkers comprising one or more amino acids, typically about 1-30, 2-24, or 3-15 amino acids. When "-" appears in a structural formula, it means that the units on both sides are directly linked by a covalent bond. When the term "bond" appears in a structural unit, it means that the unit has no amino acids and the units on both sides of the unit are directly linked.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcγ receptor (FcyR) expressed on the effector cells. For example, NK cells express FcyRIIIa, while monocytes express FcyRI, FcyRII, and FcyRIIIa. The ADCC activity of the antigen-binding molecules provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a similar manner to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding an anti-RANKL antibody or the antigen-binding molecule refers to one or more nucleic acid molecules encoding antibody heavy and light chains (or fragments thereof), including such nucleic acid molecule(s) in a single vector or separate vectors, and such nucleic acid molecule(s) present at one or more locations in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned, wherein, in the process of the optimal alignment, gaps are introduced as necessary to achieve the maximum percent sequence identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding domain and Fc domain) are linked by a covalent bond, either directly or via one or more linkers. When the linker is a peptide linker, the covalent bond is a peptide bond or amide bond. The term "vector" means a polynucleotide molecule capable of delivering another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

The term "expression vector" or "expression construct" refers to a vector that is applied to transform a host cell and comprises a nucleic acid sequence that directs and/or controls the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be exactly the same as parent cells in terms of nucleic acid content and may contain mutations. Mutant progenies that have the same function or biological activity as the cells screened or selected from the group consisting of the initially transformed cells are included herein. Host cells include prokaryotic and eukaryotic host cells, wherein eukaryotic host cells include, but are not limited to, mammalian cells, insect cells, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., HepG2), A549 cells, 3T3 cells and HEK-293 cells, *Pichiapastoris, Pichia finlandica, Candida albicans, Aspergillus niger, Aspergillus oryzae* and *Trichoderma reesei.* As used in the present application, the expressions "cell", "cell line" and "cell culture" are used interchangeably, and all such designations include progeny. Thus, the words "transformant" and "transformed cell" include the primary subject cell and cultures derived therefrom, regardless of the number of passages. It is also understood that not all progeny have precisely identical DNA contents due to deliberate or inadvertent mutations. Variant progeny that have the same function or biological activity as the original transformed cell are included.

"Optional" or "optionally" means that the event or circumstance subsequently described may, but not necessarily, occur, and that the description includes instances where the event or circumstance occurs or does not occur.

The term "pharmaceutical composition" refers to a mixture comprising one or more of the antibodies or antigen-binding molecules described herein and other chemical components, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids, such as blood; serum; serosal fluids; plasma; lymph; urine; saliva; cystic fluids; tears; excretions; sputum; mucosal secretions of secretory tissue and organs; vaginal secretions; ascites; fluids in body cavities (such as the pleura, the pericardium, the peritoneum and the abdominal cavity); fluids collected from bronchial lavage; synovial fluids; liquid solutions in contact with a subject or biological source, e.g., cell and organ culture media (including cell or organ conditioned media); lavage fluids; and the like, tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antigen-binding molecule of the present disclosure is used to delay the development of or slow the progression of disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some embodiments, the effective amount is a therapeutically effective amount or a prophylactically effective amount. "Therapeutically effective amount" is an amount sufficient to treat a disease state or condition, particularly a state or condition associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

As used herein, "pain" refers to pain of any etiology, including acute and chronic pain, and any pain with an inflammatory component. Examples of pain include, but are not limited to, post-surgical pain, post-operative pain (including dental pain), migraine, headache and trigeminal neuralgia, pain associated with burn, wound or kidney stone, pain associated with trauma (including head trauma), neuropathic pain, pain associated with musculo-skeletal disorders such as rheumatoid arthritis, osteoarthritis, ankylosing spondylitis, sero-negative (non-rheumatoid) arthropathies, non-articular rheumatism and periarticular disorders, and pain associated with cancer (including "breakthrough pain" and pain associated with terminal cancer), peripheral neuropathy and post-herpetic neuralgia. Examples of pain with an inflammatory component (in addition to those described above) include rheumatic pain, and pain associated with mucositis.

"Post-surgical pain" (interchangeably termed "post-incisional" or "post-traumatic pain") refers to pain resulting from a trauma such as a cut, puncture, incision, tear, or wound in an individual tissue (including those resulting from all surgical procedures, whether invasive or non-invasive). As used herein, post-surgical pain does not include pain that occurs (appears or arises) without an external physical trauma. In some specific embodiments, post-surgical pain is internal or external (including peripheral) pain, and the wound, cut, trauma, tear or incision may occur accidentally (with a trauma) or deliberately (with a surgical incision). As used herein, "pain" includes nociception and the sensation of pain, and pain can be assessed objectively and subjectively using pain scores and methods known in the art. Post-surgical pain, as used herein, includes allodynia (i.e., increased response to a normally non-noxious stimulus) and hyperalgesia (i.e., increased response to a normally noxious or unpleasant stimulus), which can in turn be thermal or mechanical (tactile) in nature. In some specific embodiments, the pain is characterized by thermal sensitivity, mechanical sensitivity and/or resting pain. In some specific embodiments, the post-surgical pain comprises mechanically induced pain or resting pain. In other specific embodiments, the post-surgical pain comprises resting pain. The pain is primary or secondary pain, as is well-known in the art.

### Antigen-Binding Molecules of the Present Disclosure

The present disclosure provides antigen-binding molecules having a number of advantageous properties, such as affinity, specificity for cell surface RANKL, therapeutic activity, safety, pharmacokinetic properties, and druggability (e.g., yield, purity and stability).

### Exemplary Antigen-Binding Molecules

In one aspect, the present disclosure provides an antigen-binding molecule comprising at least one first antigen-binding domain that specifically binds to RANKL and at least one second antigen-binding domain that specifically binds to NGF. Particularly, the antigen-binding molecule of the present disclosure has:
i) the first antigen-binding domain that specifically binds to RANKL binds to RANKL with a KD value of less than 5 nM, less than 4 nM, less than 3 nM, or less than 2 nM; and
   the second antigen-binding domain that specifically binds to NGF binds to NGF with a KD value of greater than 10 nM;
ii) the first antigen-binding domain that specifically binds to RANKL binds to human RANKL with an EC50 of less than 0.13 nM, and
   the second antigen-binding domain that specifically binds to NGF binds to human NGF with an EC50 of greater than 3 nM; or
iii) the first antigen-binding domain that specifically binds to RANKL blocks binding of RANKL to RANK with an IC50 of less than 0.5 nM; and
   the second antigen-binding domain that specifically binds to NGF blocks binding of NGF to TrKA with an IC50 of greater than 4 nM.

The present disclosure provides a bivalent antigen-binding molecule that specifically binds to RANKL and a bivalent antigen-binding molecule that specifically binds to NGF, which have a DVD-IgG structure.

In some embodiments, the antigen-binding molecule comprises a first chain of the structure of formula (I) and a second chain of the structure of formula (II),
(I) [RANKL-VH]-linker 1-[NGF-VH]-CH1-one subunit of Fc;
(II) [RANKL-VL]-linker 2-[NGF-VL]-CL; wherein linker 1 and linker 2 are peptide linkers identical or different in sequence.

In some embodiments, the antigen-binding molecule comprises a first chain of the structure of formula (I) and a second chain of the structure of formula (II),
(I) [NGF-VH]-linker 1-[RANKL-VH]-CH1-one subunit of Fc;
(II) [NGF-VL]-linker 2-[RANKL-VL]-CL, wherein linker 1 and linker 2 are peptide linkers identical or different in sequence.

In some embodiments, the antigen-binding molecule comprises a first chain of the structure of formula (I) and a second chain of the structure of formula (II),
(I) VHa-linker 1-VHb-CH1-one subunit of Fc;
(II) VLa-linker 2-VLb-CL;

wherein linker 1 and linker 2 are peptide linkers identical or different in sequence, and VHa comprises the sequence of SEQ ID NO: 36, 37 or 38, and VLa comprises the sequence of SEQ ID NO: 40; and
VHb comprises the sequence of SEQ ID NO: 64, and VLb comprises the sequence of SEQ ID NO: 65.

In some embodiments, the antigen-binding molecule comprises:
a first chain, comprising the sequence of SEQ ID NO: 98, 100 or 101; and
a second chain, comprising the sequence of SEQ ID NO: 99.

In some embodiments, the antigen-binding molecule comprises two first chains identical in sequence and two second chains identical in sequence.

### Variants of Antigen-Binding Molecules or Anti-RANKL Antibodies

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to arrive at the final construct, provided that the final construct possesses the desired characteristics, e.g., antigen-binding.

### Variants Comprising Substitutions, Insertions and Deletions

In certain embodiments, antigen-binding molecule variants having one or more amino acid substitutions are provided. Sites of interest include CDRs and FRs. Conservative substitutions are shown in Table 2 under the heading of "preferred substitution". More substantial changes are provided in Table 2 under the heading of "exemplary substitution", and as further described below with reference to amino acid side chain classes. Amino acid substitutions can be introduced into an antibody of interest and the products screened for a desired activity, e.g., retained/improved antigen binding, reduced immunogenicity, or improved ADCC or CDC.

**Table 2. Amino acid substitutions**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions involves substituting a member of one of these classes for a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity, reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be identified, for example, using alanine scanning mutagenesis or modeling. Particularly, HCDR3 and LCDR3 are often targeted.

In certain embodiments, substitutions, insertions or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In certain embodiments of the variant VH and VL sequences provided above, each CDR is unchanged, or contains no more than 1, 2 or 3 amino acid substitutions.

A useful method for identifying residues or regions of an antibody that can be used as mutagenesis targets is called "alanine scanning mutagenesis". In this method, a residue or group of target residues (e.g., charged residues such as Arg, Asp, His, Lys and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties. Amino acid sequence insertions include amino- and/or carboxy-terminal fusions ranging in length from 1 residue to polypeptides containing 100 or more residues, and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme or a polypeptide that increases the serum half-life of the antibody.

### Engineering of the Fc Region

In one aspect, the Fc region of the antigen-binding molecule of the present disclosure comprises one or more amino acid substitutions that reduce its binding to an Fc receptor, e.g., its binding to the Fcγ receptor, and reduce or eliminate effector functions. A natural IgG Fc region, specifically an IgG₁ Fc region or an IgG₄ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows above 50%, 80%, 90%, or 95% reduction in binding affinity for an Fc receptor as compared to the native Fc region. In some embodiments, the Fc receptor is an Fcy receptor. In some embodiments, the Fc receptor is a human Fcγ receptor, e.g., FcyRI, FcyRIIa, FcyRIIB, or FcyRIIIa. In some embodiments, the engineered Fc region also has reduced binding affinity for a complement, e.g., C1q, as compared to the native Fc region. In some embodiments, the engineered Fc region has no reduced binding affinity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG₁ Fc region, amino acid residue substitutions at positions such as positions 238, 265, 269, 270, 297, 327, and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions.

The antigen-binding molecule may comprise different antigen-binding domains fused to the two subunits of the Fc region and thus may result in undesired homodimerization. In order to improve yield and purity, it would therefore be advantageous to introduce modifications that promote heterodimerization, in the Fc region of the antigen-binding molecule of the present invention. In some embodiments, the Fc region of the present disclosure comprises engineering according to the knob-into-hole (KIH) technique, which involves introducing a protuberance structure (knob) at the interface of the first subunit and a pore structure (hole) at the interface of the second subunit. As such, the protuberance structure can be positioned in the pore structure, thereby promoting the formation of heterodimers and inhibiting the production of homodimers. The protuberance structure is constructed by substituting a small amino acid side chain at the interface of the first subunit with a larger side chain (e.g., tyrosine or tryptophan). The pore structure is created at the interface of the second subunit by substituting a large amino acid side chain with a smaller amino acid side chain (e.g., alanine or threonine). The protuberance and pore structures are prepared by altering the nucleic acid encoding the polypeptide, with optional amino acid substitutions shown in the table below:

**Table 3. Combinations of KIH mutations**

| | | | | | | | | |
|---|---|---|---|---|---|---|---|---|
| First subunit | T366Y | T366W | T394W | F405W | T366W | T366Y | T366W | F405W |
| | | | | | | F405A | F405W | Y407A |
| Second subunit | Y407T | Y407A | F405A | T394S | T366S | T394W | T394W | T366W |
| | | | | | L358A | | | |
| | | | | | | Y407T | Y407A | T394S |
| | | | | | Y407V | | | |

In addition to the knob-into-hole technique, other techniques for modifying the CH3 domain of the heavy chain of multispecific antibodies to achieve heterodimerization are also known in the art, e.g., WO96/27011, WO98/050431, EP1870459, WO2007/110205, WO 007/147901, WO2009/089004, WO2010/129304, WO2011/90754, WO2011/143545, WO2012/058768, WO2013/157954 and WO 013/096291.

The C-terminus of the Fc region may be an intact C-terminus ending with amino acid residues PGK or a shortened C-terminus, e.g., a shortened C-terminus in which one or two C-terminal amino acid residues have been removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises antibody populations having a mixture of antibodies with and without K447 residues and/or G446 + K447 residues.

### Recombination method

Antibodies can be produced using recombinant methods. For these methods, one or more isolated nucleic acids encoding the antibodies are provided.

In the case of a native antibody, a native antibody fragment or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain(s) of the antibody). These nucleic acids can be on the same expression vector or different expression vectors.

In the case of a bispecific antibody with heterodimeric heavy chains, four nucleic acids are required, one for the first light chain, one for the first heavy chain comprising the first heteromonomeric Fc-region polypeptide, one for the second light chain, and one for the second heavy chain comprising the second heteromonomeric Fc-region polypeptide. The four nucleic acids can be contained in one or more nucleic acid molecules or expression vectors. Generally, these nucleic acids are located on two or three expression vectors, that is, one vector can comprise more than one of these nucleic acids.

In one embodiment, the present disclosure provides an isolated nucleic acid encoding the antibody according to the above. Such nucleic acids may each independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule or an anti-RANKL antibody, wherein the method comprises culturing a host cell comprising a nucleic acid encoding the antibody, as provided above, under conditions suitable for antibody expression, and optionally collecting the antibody from the host cell (or host cell medium).

For recombinant production of the antigen-binding molecule or anti-RANKL antibody, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombinant methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978 and US6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

### Assays

The antigen-binding molecule or anti-RANKL antibody provided herein can be identified, screened, or characterized for its physical/chemical characteristics and/or biological activities by a variety of assays known in the art.

In one aspect, the antibody of the present disclosure is tested for antigen-binding activity, for example, by known methods such as ELISA and western blot.

In another aspect, a competition assay may be used to identify an antibody that competes for binding to RANKL. In certain embodiments, the competing antibody binds to the same epitope (e.g., a linear or conformational epitope) as the antigen-binding molecule or anti-RANKL antibody. In an exemplary competition assay, immobilized RANKL is incubated in a solution comprising a first labeled antibody that binds to RANKL and a second unlabeled antibody that is tested for its ability to compete with the first labeled antibody for binding to RANKL. The second unlabeled antibody may be present in a hybridoma supernatant. As a control, immobilized RANKL is incubated in a solution comprising the first labeled antibody but not the second unlabeled antibody. Excess unbound antibody is removed, and the amount of label associated with immobilized RANKL is measured. If the amount of label associated with immobilized RANKL is substantially reduced in the test sample relative to the control sample, then it is indicated that the second antibody competes with the first antibody for binding to RANKL.

In one aspect, an assay for identifying biologically active anti-RANKL antibodies is provided. See Test Examples of the present disclosure for details.

### Immunoconjugate

The present disclosure also provides an immunoconjugate comprising an antigen-binding molecule or an anti-RANKL antibody conjugated to one or more cytotoxic agents, such as a chemotherapeutic agent or drug, a growth inhibitor, a toxin (e.g., a protein toxin, an enzymatically active toxin, or a fragment thereof, of bacterial, fungal, plant or animal origin), or a radioisotope.

### Diagnostic and Therapeutic Compositions

In certain embodiments, the antigen-binding molecule provided by the present disclosure can be used to detect the presence of RANKL or NGF in a biological sample, and the anti-RANKL antibody provided by the present disclosure can be used to detect the presence of RANKL in a biological sample. As used herein, the term "detection" encompasses quantitative or qualitative detection. In certain embodiments, the biological sample includes a cell or tissue, such as tumor tissue.

In one embodiment, an antigen-binding molecule or anti-RANKL antibody for use in a diagnostic or detection method is provided. In yet another aspect, a method for detecting the presence of RANKL or NGF in a biological sample is provided. In certain embodiments, the method comprises contacting the biological sample with an antigen-binding molecule or an anti-RANKL antibody under suitable conditions and detecting whether a complex is formed between the detection agent and the antigen. Such methods may be *in vitro* or *in vivo* methods. In one embodiment, an antigen-binding molecule or anti-RANKL antibody is used to select a subject suitable for treatment; for example, RANKL or NGF is a biomarker for selecting a patient. Exemplary disorders that can be diagnosed using the antibody of the present disclosure are, for example, B cell disorders associated with RANKL expression, plasma cell disorders, multiple myeloma, plasmacytoma, plasma cell leukemia, macroglobulinemia, amyloidosis, Waldenström's macroglobulinaemia, solitary bone plasmacytoma, extramedullary plasmacytoma, osteosclerotic myeloma, heavy chain diseases, monoclonal gammopathy of undetermined significance, and smoldering multiple myeloma; autoimmune diseases and systemic lupus erythematosus.

In certain embodiments, a labeled antigen-binding molecule or anti-RANKL antibody is provided. Labels include, but are not limited to, labels or modules that are detected directly (such as fluorescent, chromophoric, electron-dense, chemiluminescent, and radioactive labels), and modules that are detected indirectly (e.g., modules that are detected indirectly through an enzymatic reaction or molecular interaction, such as enzymes or ligands).

In an additional aspect, a pharmaceutical composition comprising the antigen-binding molecule or anti-RANKL antibody is provided, for example, for use in any of the following treatment methods. In one aspect, the pharmaceutical composition comprises any of the antibodies provided herein and a pharmaceutically acceptable carrier. In another aspect, the pharmaceutical composition comprises any of the antibodies provided herein and at least one additional therapeutic agent.

The pharmaceutical composition of the antigen-binding molecule or anti-RANKL antibody described in the present disclosure is prepared by: mixing the antibody with one or more optional pharmaceutically acceptable carriers. The pharmaceutical composition is in the form of a lyophilized composition or an aqueous solution. Formulations for *in vivo* administration are generally sterile. Sterility can be readily achieved, for example, by filtration through a sterile filter membrane.

### Treatment method and Route of Administration

Any of the antigen-binding molecules or anti-RANKL antibodies provided herein can be used in a treatment method.

In one such embodiment, the treatment method further comprises administering to a subject an effective amount of at least one additional therapeutic agent (e.g., one, two, three, four, five, or six additional therapeutic agents). The "subject" according to any of the above embodiments may be a human.

In yet another aspect, the present disclosure provides use of the antigen-binding molecule or anti-RANKL antibody in the manufacture or preparation of a medicament. In one embodiment, the medicament is used for treating a RANKL-associated disease, such as joint stiffness or bone loss associated with RANKL expression. And the medicament is present in an amount effective for the diseases described above. In some embodiments, the effective amount is a unit daily dose or a unit weekly dose.

In yet another aspect, a pharmaceutical composition comprising the antigen-binding molecule or anti-RANKL antibody is provided, for example, for use in any of the pharmaceutical uses or treatment methods described above. In one embodiment, the pharmaceutical composition comprises any of the anti-RANKL antibodies provided herein and a pharmaceutically acceptable carrier. In another embodiment, the pharmaceutical composition further comprises at least one additional therapeutic agent. The antigen-binding molecule or anti-RANKL antibody of the present disclosure can be used alone or in combination with other agents for treatment. For example, the antibody of the present disclosure can be co-administered with at least one additional therapeutic agent.

The antigen-binding molecule or anti-RANKL antibody of the present disclosure (and any additional therapeutic agent) may be administered by any suitable means, including parenteral, intrapulmonary, and intranasal administration, and if required by local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule or anti-RANKL antibody of the present disclosure is to be formulated, administered and administered in a manner that complies with regulations or meets industry standards. Factors considered in this context include the specific disorder being treated, the specific mammal being treated, the clinical state of the individual, the cause of the disorder, the site of delivery of the agent, the method of administration, the timing of administration, and other factors known to medical practitioners. The antigen-binding molecule or anti-RANKL antibody need not be (but is optionally) formulated along with one or more agents currently used for preventing or treating the disorder. The effective amount of such additional agents depends on the amount of the antigen-binding molecule or anti-RANKL antibody present in the pharmaceutical composition, the type of the disorder or treatment, and other factors discussed above. These are generally used in the same dosages and with routes of administration as described herein, or about 1 to 99% of the dosages described herein, or in any dosage and by any route that is empirically/clinically determined to be appropriate.

For the prevention or treatment of disease, the appropriate dosage of the antigen-binding molecule or anti-RANKL antibody of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to the patient at one time or over a series of treatments. Depending on the type and severity of the disease, about 1 µg/kg to 15 mg/kg of the antigen-binding molecule or anti-RANKL antibody can be an initial candidate dosage for administration to the patient, whether, for example, by one or more separate administrations, or by continuous infusion. One typical daily dosage may range from about 1 µg/kg to 100 mg/kg or more, depending on the factors mentioned above. Accordingly, taking 50 kg body weight as an example, an exemplary unit daily dosage is 50 µg-5 g.

### Article of manufacture

In another aspect of the present disclosure, provided is an article of manufacture, which comprises materials useful for the treatment, prevention, and/or diagnosis of the above disorder. The article of manufacture comprises a container and a label or package insert on or associated with the container. Suitable containers include, for example, bottles, vials, syringes, IV solution bags, and the like. The containers may be formed from a variety of materials such as glass or plastic.

The container contains a composition which is by itself or combined with another composition effective in treating, preventing and/or diagnosing the disease, and may have a sterile access port (for example, the container may be an intravenous solution bag or a vial having a stopper pierceable by a hypodermic injection needle). At least one active agent in the composition is the antigen-binding molecule or anti-RANKL antibody of the present disclosure. The label or package insert indicates that the composition is used for treating the condition of choice.

Moreover, the article of manufacture may comprise:
(a) a first container with a composition contained therein, wherein the composition comprises the antigen-binding molecule or anti-RANKL antibody of the present disclosure; and
(b) a second container with a composition contained therein, wherein the composition comprises an additional cytotoxic or otherwise therapeutic agent.

Alternatively, or additionally, the article of manufacture may further comprise a second (or third) container comprising a pharmaceutically acceptable buffer. It may further include other materials desirable from a commercial and user standpoint, including other buffers, diluents, filters, needles and syringes.

As one example, the article of manufacture is a kit or reagent kit comprising the antigen-binding molecule or anti-RANKL antibody of the present disclosure.

### Examples and Test Examples

The present disclosure is further described below with reference to the following examples and test examples, which, however, do not limit the scope of the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: Preparation of Anti-RANKL Antibodies

Primers were designed and yeast libraries were constructed. The libraries were enriched and screened for candidate clones using the RANKL protein (Sino biological, 11682-HNCH), and the amino acid sequences of the light and heavy chain variable regions of antibodies in the clones were determined by sequencing. The sequences of the obtained exemplary anti-RANKL antibodies are as follows:

**Table 4. The CDR region sequences of antibodies**

| | | | | |
|---|---|---|---|---|
| 1-22H | HCDR1 | DYAMS SEQ ID NO: 1 | LCDR1 | RASQSVRGRYLA SEQ ID NO: 4 |
| | HCDR2 | GISASGSSTYYADSVKG SEQ ID NO: 2 | LCDR2 | GASSRAT SEQ ID NO: 5 |
| | HCDR3 | DPGTTVIMSWFDP SEQ ID NO: 3 | LCDR3 | QQYGSSPRT SEQ ID NO: 6 |
| 5-16H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPASIVIMSWFDP SEQ ID NO: 9 | LCDR3 | QQYGSSPRT SEQ ID NO: 6 |
| 5-51H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPCFSYIMSWFDP SEQ ID NO: 10 | LCDR3 | QQYGSSPRT SEQ ID NO: 6 |
| 5-53H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPCFTYIMSWFDP SEQ ID NO: 11 | LCDR3 | QQYGSSPRT SEQ ID NO: 6 |
| 5-58H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPGFTYIMSWFDP SEQ ID NO: 12 | LCDR3 | QQYGSSPRT SEQ ID NO: 6 |
| D35H | HCDR1 | AYPMS SEQ ID NO: 13 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPGTTVIMSWFDP SEQ ID NO: 3 | LCDR3 | QQYGSSPRT SEQ ID NO: 6 |
| D75H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPGTTSIMSWFDP SEQ ID NO: 14 | LCDR3 | QQYGSSPRT SEQ ID NO: 6 |
| D80H | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPGSTIIMSWFDP SEQ ID NO: 15 | LCDR3 | QQYGSSPRT SEQ ID NO: 6 |
| 2-52L | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | QGSSRAT SEQ ID NO: 16 |
| | HCDR3 | DPGTTVIMSWFDP SEQ ID NO: 3 | LCDR3 | QQYGSSPRT SEQ ID NO: 6 |
| 2-72L | HCDR1 | SYAMS SEQ ID NO: 7 | LCDR1 | RASQSLGARGLA SEQ ID NO: 17 |
| | HCDR2 | GITGSGGSTYYADSVKG SEQ ID NO: 8 | LCDR2 | GTSSRAT SEQ ID NO: 18 |
| | HCDR3 | DPGTTVIMSWFDP SEQ ID NO: 3 | LCDR3 | QQYGSSPRT SEQ ID NO: 6 |
| 2-76L | HCDR1 | SYAMS SEQ ID NO: 7 | LCDR1 | RASQAIPYNQLA SEQ ID NO: 19 |
| | HCDR2 | GITGSGGSTYYADSVKG SEQ ID NO: 8 | LCDR2 | GVSSRAT SEQ ID NO: 20 |
| | HCDR3 | DPGTTVIMSWFDP SEQ ID NO: 3 | LCDR3 | QQYGSSPRT SEQ ID NO: 6 |
| 2-79L | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GSSSRAT SEQ ID NO: 21 |
| | HCDR3 | DPGTTVIMSWFDP SEQ ID NO: 3 | LCDR3 | QQYGSSPRT SEQ ID NO: 6 |
| 2-87L | HCDR1 | SYAMS SEQ ID NO: 7 | LCDR1 | RASQYLPFHRLA SEQ ID NO: 22 |
| | HCDR2 | GITGSGGSTYYADSVKG SEQ ID NO: 8 | LCDR2 | DTSSRAT SEQ ID NO: 23 |
| | HCDR3 | DPGTTVIMSWFDP SEQ ID NO: 3 | LCDR3 | QQYGSSPRT SEQ ID NO: 6 |
| 3-20L | HCDR1 | SYAMS SEQ ID NO: 7 | LCDR1 | RASQAIRGPNLA SEQ ID NO: 24 |
| | HCDR2 | GITGSGGSTYYADSVKG SEQ ID NO: 8 | LCDR2 | DTSSRAT SEQ ID NO: 25 |
| | HCDR3 | DPGTTVIMSWFDP SEQ ID NO: 3 | LCDR3 | QQYGSSPRT SEQ ID NO: 6 |
| 3-37L | HCDR1 | SYAMS SEQ ID NO: 7 | LCDR1 | RASQAIRGRKLA SEQ ID NO: 26 |
| | HCDR2 | GITGSGGSTYYADSVKG SEQ ID NO: 8 | LCDR2 | DTSSRAT SEQ ID NO: 27 |
| | HCDR3 | DPGTTVIMSWFDP SEQ ID NO: 3 | LCDR3 | QQYGSSPRT SEQ ID NO: 6 |
| 3-83L | HCDR1 | SYAMS SEQ ID NO: 7 | LCDR1 | RASQALGVRGLA SEQ ID NO: 28 |
| | HCDR2 | GITGSGGSTYYADSVKG SEQ ID NO: 8 | LCDR2 | GASSRAT SEQ ID NO: 5 |
| | HCDR3 | DPGTTVIMSWFDP SEQ ID NO: 3 | LCDR3 | QQYGSSPRT SEQ ID NO: 6 |
| 3-85L | HCDR1 | SYAMS SEQ ID NO: 7 | LCDR1 | RASQSIPGPRLA SEQ ID NO: 29 |
| | HCDR2 | GITGSGGSTYYADSVKG SEQ ID NO: 8 | LCDR2 | GASSRAT SEQ ID NO: 5 |
| | HCDR3 | DPGTTVIMSWFDP SEQ ID NO: 3 | LCDR3 | QQYGSSPRT SEQ ID NO: 6 |
| 4-64L | HCDR1 | SYAMS SEQ ID NO: 7 | LCDR1 | RASQSVRGRYLA SEQ ID NO: 4 |
| | HCDR2 | GITGSGGSTYYADSVKG SEQ ID NO: 8 | LCDR2 | GASSRAT SEQ ID NO: 5 |
| | HCDR3 | DPGTTVIMSWFDP SEQ ID NO: 3 | LCDR3 | QQYGSSPRI SEQ ID NO: 30 |

**Table 5. The variable region sequences of RANKL antibodies**

| Antibody Name | Heavy chain variable region sequence | Light chain variable region sequence |
|---|---|---|
| 1-22H | EVQLLESGGGLVQPGGSLRLSCAASG FTFADYAMSWVRQAPGKGLEWVSGI SASGSSTYYADSVKGRFTISRDNSKNT LYLQMNSLRAEDTAVYYCAKDPGTT VIMSWFDPWGQGTLVTVSS SEQ ID NO: 31 | EIVLTQSPGTLSLSPGERA TLSCRASQSVRGRYLAW YQQKPGQAPRLLIYGASS RATGIPDRFSGSGSGTDFT LTISRLEPEDFAVFYCQQY GSSPRTFGQGTKVEIK SEQ ID NO: 40 |
| 5-16H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCAKDPASIVI MSWFDPWGQGTLVTVSS SEQ ID NO: 32 | |
| 5-51H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCAKDPCFSYI MSWFDPWGQGTLVTVSS SEQ ID NO: 33 | |
| 5-53H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCAQDPCFTYI MSWFDPWGQGTLVTVSS SEQ ID NO: 34 | |
| 5-58H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCVQDPGFTYI MSWFDPWGQGTLVTVSS SEQ ID NO: 35 | |
| D35H | EVQLLESGGGLVQPGGSLRLSCAASG FTFSAYPMSWVRQAPGKGLEWVSGIT GSGGSTYYADSVKGRFTISRDNSKNTL YLQMNSLRAEDTAVYYCAKDPGTTVI MSWFDPWGQGTLVTVSS SEQ ID NO: 36 | |
| D75H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCVKDPGTTSI MSWFDPWGQGTLVTVSS SEQ ID NO: 37 | |
| D80H | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCVKDPGSTII MSWFDPWGQGTLVTVSS SEQ ID NO: 38 | |
| 2-52L | EVQLLESGGGLVQPGGSLRLSCAASG FTFSSYAMSWVRQAPGKGLEWVSGIT GSGGSTYYADSVKGRFTISRDNSKNTL YLQMNSLRAEDTAVYYCAKDPGTTVI MSWFDPWGQGTLVTVSS SEQ ID NO: 39 | EIVLTQSPGTLSLSPGERA TLSCRASQSVRGRYLAW YQQKPGQAPRLLIYQGSS RATGIPDRFSGSGSGTDFT LTISRLEPEDFAVFYCQQY GSSPRTFGQGTKVEIK SEQ ID NO: 41 |
| 2-72L | | EIVLTQSPGTLSLSPGERA TLSCRASQSLGARGLAW YQQKPGQAPRLLIYGTSS RATGIPDRFSGSGSGTDFT LTISRLEPEDFAVFYCQQY GSSPRTFGQGTKVEIK SEQ ID NO: 42 |
| 2-76L | | EIVLTQSPGTLSLSPGERA TLSCRASQAIPYNQLAWY QQKPGQAPRLLIYGVSSR ATGIPDRFSGSGSGTDFTL TISRLEPEDFAVFYCQQYG SSPRTFGQGTKVEIK SEQ ID NO: 43 |
| 2-79L | | EIVLTQSPGTLSLSPGERA TLSCRASQSVRGRYLAW YQQKPGQAPRLLIYGSSS RATGIPDRFSGSGSGTDFT LTISRLEPEDFAVFYCQQY GSSPRTFGQGTKVEIK SEQ ID NO: 44 |
| 2-87L | | EIVLTQSPGTLSLSPGERA TLSCRASQYLPFHRLAWY QQKPGQAPRLLIYDTSSR ATGIPDRFSGSGSGTDFTL TISRLEPEDFAVFYCQQYG SSPRTFGQGTKVEIK SEQ ID NO: 45 |
| 3-20L | | EIVLTQSPGTLSLSPGERA TLSCRASQAIRGPNLAWY QQKPGQAPRLLIYDTSSR ATGIPDRFSGSGSGTDFTL TISRLEPEDFAVFYCQQYG SSPRTFGQGTKVEIK SEQ ID NO: 46 |
| 3-37L | | EIVLTQSPGTLSLSPGERA TLSCRASQAIRGRKLAWY QQKPGQAPRLLIYDTSSR ATGIPDRFSGSGSGTDFTL TISRLEPEDFAVFYCQQYG SSPRTFGQGTKVEIK SEQ ID NO: 47 |
| 3-83L | | EIVLTQSPGTLSLSPGERA TLSCRASQALGVRGLAW YQQKPGQAPRLLIYGASS RATGIPDRFSGSGSGTDFT LTISRLEPEDFAVFYCQQY GSSPRTFGQGTKVEIK SEQ ID NO: 48 |
| 3-85L | | EIVLTQSPGTLSLSPGERA TLSCRASQSIPGPRLAWY QQKPGQAPRLLIYGASSR ATGIPDRFSGSGSGTDFTL TISRLEPEDFAVFYCQQYG SSPRTFGQGTKVEIK SEQ ID NO: 49 |
| 4-64L | | EIVLTQSPGTLSLSPGERA TLSCRASQSVRGRYLAW YQQKPGQAPRLLIYGASS RATGIPDRFSGSGSGTDFT LTISRLEPEDFAVFYCQQY GSSPRIFGQGTKVEIK SEQ ID NO: 50 |

The above variable regions were fused to a human light chain constant region and a heavy chain constant region to form complete antibody light and heavy chains. The constant region sequences of exemplary antibodies are as follows:
Human IgG4 heavy chain constant region: 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
SEQ ID NO: 51;
Human κ light chain constant region:



SEQ ID NO: 52.

**The sequences of exemplary anti-RANKL antibodies are as follows:**
**D35H heavy chain:** 
EVQLLESGGGLVQPGGSLRLSCAASGFTFSAYPMSWVRQAPGKGLEWVSGITG
SGGSTYYADSVKGRFTISRDNSKNTLYLQMNSLRAEDTAVYYCAKDPGTTVIM
SWFDPWGQGTLVTVSSASTKGPSVFPLAPCSRSTSESTAALGCLVKDYFPEPVTVS
WNSGALTSGVHTFPAVLQSSGLYSLSSVVTVPSSSLGTKTYTCNVDHKPSNTKVDKRV
ESKYGPPCPPCPAPEFLGGPSVFLFPPKPKDTLMISRTPEVTCVVVDVSQEDPEVQ
FNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQ
PENNYKTTPPVLDSDGSFFLYSRLTVDKSRWQEGNVFSCSVMHEALHNHYTQKSLS
LSLGK
SEQ ID NO: 53;
**D35H light chain:** 
EIVLTQSPGTLSLSPGERATLSCRASQSVRGYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTEQDS
 
KDSTYSLSSTLTLSKADYEKHKVYACEVTHQGLSSPVTKSFNRGEC
SEQ ID NO: 54.
**D75H heavy chain:** 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGTTSIM
SWFDPWGQGTLVTVSSASTKGPSVFPLAPCSRSTSESTAALGCLVKDYFPEPVTVS
WNSGALTSGVHTFPAVLQSSGLYSLSSVVTVPSSSLGTKTYTCNVDHKPSNTKVDKRV
ESKYGPPCPPCPAPEFLGGPSVFLFPPKPKDTLMISRTPEVTCVVVDVSQEDPEVQ
FNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQ
PENNYKTTPPVLDSDGSFFLYSRLTVDKSRWQEGNVFSCSVMHEALHNHYTQKSLS
LSLGK
SEQ ID NO: 55;
**D75H light chain:** 
EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 54.
**D80H heavy chain:** 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGSTIIMS
WFDPWGQGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDKRVE
SKYGPPCPPCPAPEFLGGPSVFLFPPKPKDTLMISRTPEVTCVVVDVSQEDPEVQF
NWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQP
ENNYKTTPPVLDSDGSFFLYSRLTVDKSRWQEGNVFSCSVMHEALHNHYTQKSLSL
SLGK
SEQ ID NO: 56;
**D80H light chain;**

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 54.

The sequences of a control anti-RANKL antibody are as follows:
Denosumab heavy chain sequence: 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCAKDPGTTVIMS
WFDPWGQGTLVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKV
DKTVERKCCVECPPCPAPPVAGPSVFLFPPKPKDTLMISRTPEVTCVVVDVSHED
PEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEA
LHNHYTQKSLSLSPGK
SEQ ID NO: 57;
Denosumab light chain sequence:

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 54.

### Example 2: Preparation of Anti-RANKL/NGF Bispecific Antibodies

The second antigen-binding domains of the bispecific antibodies of the present disclosure that bind to NGF can be derived from the antigen-binding moiety of any suitable antibody. Particularly suitable antibodies are described, for example, in the international application WO2004058184 (herein incorporated by reference in its entirety).

The NGF binding domain CDRs and variable region sequences of exemplary bispecific antibodies are shown below:

**Table 6. The CDRs of the NGF binding domain**

| | | | |
|---|---|---|---|
| HCDR1 | GYDLN (SEQ ID NO: 58) | LCDR1 | RASQSISNNLN (SEQ ID NO: 61) |
| HCDR2 | IIWGDGTTDYNSAVKS (SEQ ID NO: 59) | LCDR2 | YTSRFHS (SEQ ID NO: 62) |
| HCDR3 | GGYWYATSYYFDY (SEQ ID NO: 60) | LCDR3 | QQEHTLPYT (SEQ ID NO: 63) |

NGF binding domain heavy chain variable region: 



SEQ ID NO: 64;
NGF binding domain light chain variable region:




SEQ ID NO: 65.

The variable regions of the NGF binding domain were fused to a heavy chain constant region of SEQ ID NO: 51 or SEQ ID NO: 66 and a light chain constant region of SEQ ID NO: 52, respectively, to obtain an anti-NGF antibody. The sequences are as follows: 
ASTKGPSVFPLAPCSRSTSESTAALGCLVKDYFPEPVTVSWNSGALTSGVHTFPA
VLQSSGLYSLSSVVTVPSSNFGTQTYTCNVDHKPSNTKVDKTVERKCCVECPPC
PAPPVAGPSVFLFPPKPKDTLMISRTPEVTCVVVDVSHEDPEVQFNWYVDGVEV
HNAKTKPREEQFNSTFRVVSVLTVVHQDWLNGKEYKCKVSNKGLPSSIEKTISK
TKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSP
GK
SEQ ID NO: 66;
anti-NGF antibody (N-mAb) heavy chain: 
QVQLQESGPGLVKPSETLSLTCTVSGFSLIGYDLNWIRQPPGKGLEWIGIIWGDG
TTDYNSAVKSRVTISKDTSKNQFSLKLSSVTAADTAVYYCARGGYWYATSYYFD
YWGQGTLVTVSSASTKGPSVFPLAPCSRSTSESTAALGCLVKDYFPEPVTVSWNSGA
LTSGVHTFPAVLQSSGLYSLSSVVTVPSSSLGTKTYTCNVDHKPSNTKVDKRVESKYGP
PCPPCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK
SEQ ID NO: 67;
anti-NGF antibody (N-mAb) light chain:

DIQMTQSPSSLSASVGDRVTITCRASQSISNNLNWYQQKPGKAPKLLIYYTSRFH
SGVPSRFSGSGSGTDFTFTISSLQPEDIATYYCQQEHTLPYTFGQGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO: 68.

The sequences of the control anti-NGF antibody:
Tanezumab heavy chain: 
QVQLQESGPGLVKPSETLSLTCTVSGFSLIGYDLNWIRQPPGKGLEWIGIIWGDG
TTDYNSAVKSRVTISKDTSKNQFSLKLSSVTAADTAVYYCARGGYWYATSYYFD
YWGQGTLVTVSSASTKGPSVFPLAPCSRSTSESTAALGCLVKDYFPEPVTVSWNSGA
LTSGVHTFPAVLQSSGLYSLSSVVTVPSSNFGTQTYTCNVDHKPSNTKVDKTVERKCC
VECPPCPAPPVAGPSVFLFPPKPKDTLMISRTPEVTCVVVDVSHEDPEVQFNWYVDG
VEVHNAKTKPREEQFNSTFRVVSVLTVVHQDWLNGKEYKCKVSNKGLPSSIEKTISKT
KGQPREPQVYTLPPSREEMTKNQVSLTCLVKGFYPSDIAVEWESNGQPENNYKTTPP
MLDSDGSFFLYSKLTVDKSRWQQGNVFSCSVMHEALHNHYTQKSLSLSPGK
SEQ ID NO: 69;
>Tanezumab light chain:

DIQMTQSPSSLSASVGDRVTITCRASQSISNNLNWYQQKPGKAPKLLIYYTSRFH
SGVPSRFSGSGSGTDFTFTISSLQPEDIATYYCQQEHTLPYTFGQGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO: 68.

For the bone metastasis indication, monoclonal antibodies targeting NGF differ considerably from those targeting RANKL in clinical dosage at present. Therefore, in preparing a bispecific antibody against NGF and RANKL, it is necessary to balance the activities of the NGF arm and RANKL arm of the bispecific antibody so as to determine an appropriate dosage of the bispecific antibody, which will not cause the side effects arising from excessively high dosages or fail to fulfill the function of the bispecific antibody as excessively low dosages will.

In the present disclosure, a variety of structurally distinct bispecific antibodies were prepared, and by way of example, bispecific antibodies with a DVD-IgG structure were constructed. Two different light chain variable domains (VL) from an anti-RANKL antibody and an anti-NGF antibody were linked in tandem, directly or via a short linker by recombinant DNA techniques, followed by the light chain constant domain. Similarly, the heavy chain comprises two different heavy chain variable domains (VH) linked in tandem, followed by the constant domain CH1 and Fc region. The linker includes, but is not limited to, the following peptide linkers: ASTKGP (SEQ ID NO: 70), TVAAPS (SEQ ID NO: 71), ASTKGPSVFPLAP (SEQ ID NO: 72), TVAAPSVFIFPP (SEQ ID NO: 73), AKTTPKLEEGEFSEAR (SEQ ID NO: 74), AKTTPKLEEGEFSEARV (SEQ ID NO: 75), AKTTPKLGG (SEQ ID NO: 76), SAKTTPKLGG (SEQ ID NO: 77), SAKTTP (SEQ ID NO: 78), RADAAP (SEQ ID NO: 79), RADAAPTVS (SEQ ID NO: 80), RADAAAAGGPGS (SEQ ID NO: 81), SAKTTPKLEEGEFSEARV (SEQ ID NO: 82), ADAAP (SEQ ID NO: 83), ADAAPTVSIFPP (SEQ ID NO: 84), TVAAP (SEQ ID NO: 85), TVAAPSVFIFPP (SEQ ID NO: 86), QPKAAP (SEQ ID NO: 87), QPKAAPSVTLFPP (SEQ ID NO: 88), AKTTPP (SEQ ID NO: 89), AKTTPPSVTPLAP (SEQ ID NO: 90), AKTTAP (SEQ ID NO: 91), AKTTAPSVYPLAP (SEQ ID NO: 92), ASTKGPSVFPLAP (SEQ ID NO: 93), GGGGSGGGGSGGGGS (SEQ ID NO: 94), GENKVEYAPALMALS (SEQ ID NO: 95), GPAKELTPLKEAKVS (SEQ ID NO: 96) and GHEAAAVMQVQYPAS (SEQ ID NO: 97).

The sequences of exemplary bispecific antibodies are as follows:
>First chain of bispecific antibody 1 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGTTSIMS
WFDPWGQGTLVTVSSASTKGPQVQLQESGPGLVKPSETLSLTCTVSGFSLIGYD
LNWIRQPPGKGLEWIGIIWGDGTTDYNSAVKSRVTISKDTSKNQFSLKLSSVTAA
DTAVYYCARGGYWYATSYYFDYWGQGTLVTVSSASTKGPSVFPLAPCSRSTSES
 
TAALGCLVKDYFPEPVTVSWNSGALTSGVHTFPAVLQSSGLYSLSSVVTVPSSSL
GTKTYTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGK
SEQ ID NO: 98;
>second chain of bispecific antibody 1 
EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKTVA
APDIQMTQSPSSLSASVGDRVTITCRASQSISNNLNWYQQKPGKAPKLLIYYTSR
FHSGVPSRFSGSGSGTDFTFTISSLQPEDIATYYCQQEHTLPYTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 99.
First chain of bispecific antibody 2: 
EVQLLESGGGLVQPGGSLRLSCAASGFTFSAYPMSWVRQAPGKGLEWVSGITG
SGGSTYYADSVKGRFTISRDNSKNTLYLQMNSLRAEDTAVYYCAKDPGTTVIMS
WFDPWGQGTLVTVSSASTKGPQVQLQESGPGLVKPSETLSLTCTVSGFSLIGYD
LNWIRQPPGKGLEWIGIIWGDGTTDYNSAVKSRVTISKDTSKNQFSLKLSSVTAA
DTAVYYCARGGYWYATSYYFDYWGQGTLVTVSSASTKGPSVFPLAPCSRSTSES
TAALGCLVKDYFPEPVTVSWNSGALTSGVHTFPAVLQSSGLYSLSSVVTVPSSSL
GTKTYTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGK
SEQ ID NO: 100;
second chain of bispecific antibody 2: 
EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKTVA
APDIQMTQSPSSLSASVGDRVTITCRASQSISNNLNWYQQKPGKAPKLLIYYTSR
FHSGVPSRFSGSGSGTDFTFTISSLQPEDIATYYCQQEHTLPYTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 99.
First chain of bispecific antibody 3 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGSTIIMS
 
WFDPWGQGTLVTVSSASTKGPQVQLQESGPGLVKPSETLSLTCTVSGFSLIGYD
LNWIRQPPGKGLEWIGIIWGDGTTDYNSAVKSRVTISKDTSKNQFSLKLSSVTAA
DTAVYYCARGGYWYATSYYFDYWGQGTLVTVSSASTKGPSVFPLAPCSRSTSES
TAALGCLVKDYFPEPVTVSWNSGALTSGVHTFPAVLQSSGLYSLSSVVTVPSSSL
GTKTYTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGK
SEQ ID NO: 101;
second chain of bispecific antibody 2:

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKTVA
APDIQMTQSPSSLSASVGDRVTITCRASQSISNNLNWYQQKPGKAPKLLIYYTSR
FHSGVPSRFSGSGSGTDFTFTISSLQPEDIATYYCQQEHTLPYTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 99.

Note: The sequences single-underlined are RANKL antibody variable regions, the sequences double-underlined are NGF antibody variable regions, the sequences in bold type are linkers, and the others are constant regions.

### Test Examples

### Test Example 1: Tests of Anti-RANKL Antibodies for Affinity

A biosensor chip Protein A (GE, 29127556) was used to affinity-capture a certain amount of test antibody, and then antigens, the human RANKL protein (Sino Biological, 11682-HNCH) and the human NGF protein (Sino biological, 11050-HNAC), at a series of concentrations were allowed to flow over the surface of the chip. Reaction signals were detected in real time by using Biacore, and binding and dissociation curves were obtained. After dissociation was complete in each cycle, the biochip was washed and regenerated with a glycine-hydrochloric acid regeneration solution (GE, BR-1003-54) having a pH of 1.5. Fitting was performed on the experimental data using BIAevaluation version 4.1 software with a 1:1 model, and affinity values were obtained. The results are shown in Table 7.

**Table 7. The affinity KD values of anti-RANKL antibodies**

| Antibody | Affinity for human RANKL | | | |
|---|---|---|---|---|
| | ka (1/Ms) | kd (1/s) | KD (M) | Ratio of affinities (fold increase in affinity relative to denosumab) |
| 1-22H | 2.58E+04 | 2.53E-04 | 9.81E-09 | 0.94 |
| 5-16H | 2.92E+04 | 2.60E-04 | 8.91E-09 | 1.04 |
| 5-51H | 2.92E+04 | 6.90E-04 | 2.36E-08 | 0.39 |
| 5-53H | 1.72E+04 | 5.83E-04 | 3.40E-08 | 0.27 |
| 5-58H | 1.22E+04 | 5.01E-04 | 4.10E-08 | 0.23 |
| D35H | 2.71E+04 | 7.61E-05 | 2.81E-09 | **3.29** |
| D75H | 5.24E+04 | 7.73E-05 | 1.48E-09 | **6.24** |
| D80H | 1.97E+04 | 7.40E-05 | 3.76E-09 | **2.46** |
| 2-52L | 1.57E+04 | 2.22E-04 | 1.42E-08 | 0.65 |
| 2-72L | 1.62E+04 | 3.08E-04 | 1.90E-08 | 0.49 |
| 2-76L | 1.68E+04 | 3.41E-04 | 2.03E-08 | 0.46 |
| 2-79L | 1.42E+04 | 2.32E-04 | 1.63E-08 | 0.57 |
| 2-87L | 1.07E+04 | 2.73E-04 | 2.56E-08 | 0.36 |
| 3-20L | 2.01E+04 | 2.72E-04 | 1.35E-08 | 0.68 |
| 3-37L | 1.55E+04 | 3.05E-04 | 1.97E-08 | 0.47 |
| 3-83L | 1.56E+04 | 3.22E-04 | 2.07E-08 | 0.45 |
| 3-85L | 1.50E+04 | 3.35E-04 | 2.23E-08 | 0.41 |
| 4-64L | 1.48E+04 | 2.53E-04 | 1.71E-08 | 0.54 |
| Denosumab | 1.90E+04 | 1.75E-04 | 9.24E-09 | 1.00 |

The results show that the affinity of the engineered antibody D75H is more than 6 times higher than that of denosumab, and the affinities of D35H and D80H are also significantly better than that of denosumab. The affinities of the other antibodies for RANKL are not significantly higher. They are even lower than that of denosumab.

### Test Example 2: Tests of Anti-RANKL Antibodies for Abilities to Block Binding of RANKL to RANK

The activity of antibodies blocking binding of the ligand to the receptor was determined by ELISA. The human RANK protein was diluted to 2 µg/mL with pH 7.4 PBS (BasalMedia, B320) and added to a 96-well microplate (Corning, 3590) at 100 µL/well, and the plate was incubated overnight at 4 °C. After the liquid was discarded, 200 µL of 1% Casein blocking solution (Thermo, 37528) was added to each well for blocking, and the plate was incubated at 37 °C for 2 h. After the blocking was complete, the blocking solution was discarded, and the plate was washed 3 times with a PBST buffer (pH 7.4 PBS containing 0.1% tween-20) for later use. After a fixed concentration of biotin-labeled human RANKL protein (Sino Biological, 11682-HNCH) was mixed with an antibody serially diluted, the mixtures were pre-incubated at 37 °C for 30 min, then added to the blocked microplate, and incubated at 37 °C for 1.5 h. After the incubation was complete, the plate was washed 3 times with PBST. Streptavidin-HRP (Invitrogen, 434323, diluted at 1:4000) was added at 100 µL/well, and the plate was incubated at 37 °C for 1 h. The supernatant was removed. After the plate was washed 3 times with PBST, TMB chromogenic substrate (KPL, 5120-0077) was added at 100 µL/well, and the plate was incubated at room temperature for 10 to 15 min. 1 M H₂SO₄ was added at 50 µL/well to stop the reactions. The absorbance values at 450 nm were taken using a microplate reader. A curve for the inhibition of the binding of the ligand to the receptor was fit using software, and an IC50 value was calculated. The results are shown in Table 8.

**Table 8. The abilities of anti-RANKL antibodies to block binding of RANKL to RANK**

| Name of antibody | Blocking binding of RANKL to RANK IC50 (nM) |
|---|---|
| 5-53H | 1.366 |
| 5-58H | 0.9992 |
| D35H | **0.2891** |
| D75H | **0.2964** |
| D80H | **0.3659** |
| Denosumab | 0.5324 |

The results show that the anti-RANKL antibodies D35H, D75H and D80H can all block binding of RANKL to RANK, and their blocking activity is better than that of denosumab.

### Test Example 3: Determination of Binding Activity of Bispecific Antibodies for Antigens of Different Species by ELISA

The binding activity of the bispecific antibodies of the present disclosure for human RANKL (Sino biological, 11682-HNCH), monkey RANKL (Sino Biological, 90301-C01H), murine RANKL (R&D Systems, 462-TR/CF) and human NGF (the monkey NGF and the human NGF are identical in sequence) (Sino biological, 11050-HNAC), and murine NGF (Sino Biological, 50385-MNAC) was determined by ELISA as follows:
The antigen was diluted to 1 µg/mL with a pH 7.4 PBS buffer (BasalMedia, B320) and added to a 96-well microplate (Corning, 9018) at 100 µL/well, and the plate was incubated overnight at 4 °C. After the liquid was discarded, a 5% skim milk (BD, 232100) blocking solution diluted with PBS was added at 300 µL/well, and the plate was incubated at 37 °C for 2 h for blocking. After the blocking was complete, the blocking solution was discarded, and the plate was washed 3 times with a PBST buffer (pH 7.4 PBS containing 0.1% tween-20). Then a bispecific antibody diluted with a sample diluent (pH 7.4 containing 1% BSA) to different concentrations was added at 100 µL/well, and the plate was incubated in a 37 °C incubator for 1 h. After the incubation was complete, the plate was washed 3 times with PBST. An HRP-labeled anti-human Fc secondary antibody (Abcam, ab97225) diluted with the sample diluent was added at 100 µL/well, and the plate was incubated at 37 °C for 1 h. After the plate was washed 3 times with PBST, TMB chromogenic substrate (KPL, 5120-0077) was added at 100 µL/well, and the plate was incubated at room temperature for 10 to 15 min. 50 µL of 1 M H₂SO₄ was added to each well to stop the reactions. The absorbance values at 450 nm were taken using a microplate reader, and the EC50 values for binding of the bispecific antibody to the antigens were calculated. The results are shown in Table 9.

**Table 9. EC50 for binding of bispecific antibodies to antigens of different species**

| Antibody | ELISA EC50 (nM) | | | | |
|---|---|---|---|---|---|
| | Human RANKL | Monkey RANKL | Murine RANKL | Human (monkey) NGF | Murine NGF |
| Bispecific antibody 1 | 0.06245 | 0.4666 | No binding | 12.27 | 4.344 |
| Denosumab | 0.1277 | 2.129 | No binding | No binding | No binding |
| Tanezumab | No binding | No binding | No binding | 2.270 | 1.151 |

The results show that the binding activity of bispecific antibody 1 for whether human RANKL or monkey RANKL is better than that of the control antibody denosumab. However, neither bispecific antibody 1 nor denosumab cross-bound to murine RANKL. The binding activity of bispecific antibody 1 for whether human NGF or murine NGF is significantly weaker than that of tanezumab, which indicates that bispecific antibody 1 of the present disclosure had successfully reduced the binding activity of the tanezumab terminus (the second antigen-binding domain that specifically binds to NGF).

### Test Example 4: Tests of Bispecific Antibodies for Affinity

A biosensor chip Protein A (GE, 29127556) was used to affinity-capture a certain amount of test antibody, and then antigens, the human RANKL protein (Sino Biological, 11682-HNCH) and the human NGF protein (Sino biological, 11050-HNAC), at a series of concentrations were allowed to flow over the surface of the chip. Reaction signals were detected in real time by using Biacore, and binding and dissociation curves were obtained. After dissociation was complete in each cycle, the biochip was washed and regenerated with a glycine-hydrochloric acid regeneration solution (GE, BR-1003-54) having a pH of 1.5. Fitting was performed on the experimental data using BIAevaluation version 4.1 software with a 1:1 model, and affinity values were obtained. The results are shown in Table 10.

**Table 10. Affinities of bispecific antibodies for different antigens**

| Antibody | Affinity for human RANKL | | | Affinity for human NGF | | |
|---|---|---|---|---|---|---|
| | ka (1/Ms) | kd (1/s) | KD (M) | ka (1/Ms) | kd (1/s) | KD (M) |
| Bispecific antibody 1 | 2.25E+04 | 8.77E-05 | 3.89E-09 | 1.13E+03 | 6.33E-05 | 5.61E-08 |
| Denosumab | 1.04E+04 | 1.53E-04 | 1.47E-08 | / | / | / |
| Tanezumab | / | / | / | 1.29E+06 | 1.84E-04 | 1.42E-10 |

The results show that the affinity of bispecific antibody 1 for human RANKL is 3.8 times that of denosumab, and the affinity of bispecific antibody 1 for human NGF is significantly lower than that of tanezumab.

### Test Example 5: Blocking Experiments of Ligands and Receptors

The blocking activity of antibodies for ligands and receptors is determined by ELISA. A receptor protein (RANK or TrkA) was diluted to 2 µg/mL with pH 7.4 PBS (BasalMedia, B320) and added to a 96-well microplate (Corning, 3590) at 100 µL/well, and the plate was incubated overnight at 4 °C. After the liquid was discarded, 200 µL of 1% Casein blocking solution (Thermo, 37528) was added to each well for blocking, and the plate was incubated at 37 °C for 2 h. After the blocking was complete, the blocking solution was discarded, and the plate was washed 3 times with a PBST buffer (pH 7.4 PBS containing 0.1% tween-20) for later use. After a fixed concentration of biotin-labeled ligand protein (RANKL or NGF) was mixed with an antibody or fusion protein serially diluted, the mixtures were pre-incubated at 37 °C for 30 min and then added to the blocked microplate. The plate was incubated at 37 °C for 1.5 h. After the incubation was complete, the plate was washed 3 times with PBST. Streptavidin-HRP (Invitrogen, 434323, diluted at 1:4000) was added at 100 µL/well, and the plate was incubated at 37 °C for 1 h. The supernatant was removed. After the plate was washed 3 times with PBST, TMB chromogenic substrate (KPL, 5120-0077) was added at 100 µL/well, and the plate was incubated at room temperature for 10 to 15 min. 1 M H₂SO₄ was added at 50 µL/well to stop the reactions. The absorbance values at 450 nm were taken using a microplate reader. A curve for the inhibition of binding of the ligand to the receptor was fit using software, and an IC50 value was calculated.

The ligand and receptor proteins used in the experiments are as follows: human RANKL protein (Sino Biological, 11682-HNCH), human RANK protein (Sino Biological, 16078-H02H), human TrkA protein (Sino Biological, 11073-H03H), and human NGF protein (Sino biological, 11050-HNAC).

The experimental results are shown in Table 11.

**Table 11. The blocking activity of bispecific antibodies**

| Antibody | Blocking binding of RANKL to RANK | Blocking binding of NGF to TrkA |
|---|---|---|
| | IC50 (nM) | IC50 (nM) |
| Bispecific antibody 1 | 0.3225 | 102.1 |
| Denosumab | 0.5585 | / |
| Tanezumab | / | 3.663 |

The results show that the blocking activity of bispecific antibody 1 for RANKL is better than that of denosumab, and its blocking activity for NGF is significantly weaker than that of tanezumab. This indicates that reducing the binding activity of the NGF terminus of bispecific antibody 1 also significantly reduced its blocking activity for NGF.

### Test Example 6: Osteoclast Differentiation Experiments

The major function of RANKL is to promote the differentiation and maturation of osteoclasts. Therefore, the inhibitory activity of antibodies against RANKL can be tested by osteoclast differentiation experiments. The specific procedures were as follows:
Raw264.7 cells (ECACC, 91062702) were plated in a 96-well cell culture plate (Corning, 3599) and incubated overnight in a 37 °C incubator. The next day, a fixed concentration of the human RANKL protein (Sino Biological, 11682-HNCH) and a serially diluted test bispecific antibody were added to the 96-well cell plate, and the plate was incubated at 37 °C for 4 days. The 96-well plate was taken out, and the supernatants were removed. A cell lysis solution (Beyotime, P0013J) was added at 100 µL/well. After the mixtures were well mixed using a pipette, the cell lysates were transferred to centrifuged tubes, placed on ice for 10 min and centrifuged, and the supernatants were collected. Referring to the method described in the tartaric acid-resistant acidic phosphatase assay kit (Beyotime, P0332), 40 µL of a test sample was taken and well mixed with 40 µL of a chromogenic substrate and 5 µL of tartaric acid; the mixture was incubated in a 37 °C incubator for 10 min, and 160 µL of stop solution was added; the values of OD 405nm were taken using a microplate reader, fitting was performed on the data using software, and an IC50 value was obtained. The experimental results are shown in Table 12.

**Table 12. The osteoclast differentiation-inhibiting activity of bispecific antibodies**

| Antibody | IC50 (nM) |
|---|---|
| Bispecific antibody 1 | 1.193 |
| Denosumab | 3.917 |

The results show that the osteoclast differentiation-inhibiting activity of bispecific antibody 1 is more than 3.3 times that of denosumab.

### Test Example 7: TF-1 Cell Proliferation Experiment

NGF promotes the proliferation of TF-1 cells *in vitro.* Therefore, the inhibitory activity of antibodies against NGF was assessed by TF-1 cell proliferation experiments. The specific procedures were as follows:
TF-1 cells (ATCC, CRL-2003) were resuspended in a GM-CSF-free 1640 medium (Gibco, 22400-105) and plated in a 96-well cell culture plate (Corning, 3903), and the plate was incubated in a 37 °C incubator overnight. The next day, a fixed concentration of the human NGF protein (Sino biological, 11050-HNAC) and a serially diluted test antibody were added to the 96-well cell plate, and the plate was incubated in a 37 °C incubator for 72 h. The cell culture plate was taken out, and Cell-titer Glo (Promega, G755B) solution was added at 50 µL/well. The plate was gently shaken for 10 min and left to stand at room temperature for 10 min, and bioluminescent signals were detected using PE Victor 3. Fitting was performed on data using software, and an IC50 value was obtained. The experimental results are shown in Table 13.

**Table 13. The TF-1 cell proliferation-inhibiting activity of bispecific antibodies**

| Antibody | IC50 (nM) |
|---|---|
| Bispecific antibody 1 | 3.159 |
| Tanezumab | 0.7589 |

The results show that bispecific antibody 1 can still inhibit TF-1 cell proliferation, and the inhibitory activity of tanezumab is about 4.2 times that of bispecific antibody 1, which indicates that the activity of the NGF terminus of bispecific antibody 1 was significantly reduced.

Therefore, on condition that there is a correlation between *in vitro* activity and dosage, we expect that the dosage of the RANKL terminus of bispecific antibody 1 can be reduced to about 40 mg, and the in vivo efficacy of the RANKL terminus can still be maintained; the dosage of the NGF terminus can be increased to about 80 mg, and the good safety can still be maintained. In addition, the clinical administration cycle of the antibody tanezumab against NGF is 2 times that of the antibody denosumab against RANKL at present. Therefore, the exemplary bispecific antibody 1 of the present disclosure can balance the dosages for the two targets.

### Test Example 8: In Vivo Efficacy in Animal Model of Bone Metastasis

The analgesic and bone-protecting effects of antibodies were assessed using an animal model of bone metastasis.

A construction method of the model: male C57 BL/6 mice, SPF, were purchased from Changzhou Cavens Laboratory Animal Co., Ltd. The animals were acclimatized to the laboratory environment, a 12/12-hour light/dark cycle was adopted, the temperature was 23±1 °C, and the humidity was 40 to 50%. The animals were given *ad libitum* access to standard sterile mouse feed and water. When the body weight of the mice reached about 25 g, modeling was started.

After 1% sodium barbiturate was intraperitoneally injected to anesthetize a mouse, the skin was prepared. The mouse was placed on a heating pad, a 1-cm cut was made, with ophthalmological scissors, in the skin on the lateral part of the knee of the left hind limb, in a direction parallel to the femur. The muscles were exposed, and the skin and muscle layers were isolated bluntly. An incision was made, with ophthalmological scissors, between rectus femoris and vastus medialis, along the lines in connective tissue. Rectus femoris and the patella were moved to the medial side of the knee joint with curved forceps. The femoral condyle was exposed without cutting off the patellar ligament. An opening was made, with a needle with a 0.45-mm diameter, from the top to the middle of the femur at the intercondylar fossa. The needle was inserted into the intramedullary space by 1 to 1.5 cm to form an injection channel. A Hamilton microsyringe (50 µL, model 1705 RN SYR, 26 gauge needle, model ga26/51 mm/pst3, catalog No. 7768-02) was inserted into the marrow cavity through the opening and 10 µL (5 × 104) of LLC1 cells (ATCC, CRL-1642) was slowly injected. After the injection was complete, the syringe needle was pulled out. The leg was straightened, the patella and ligament were reset with curved forceps, and the muscles were restored to their original states. An antibiotic powder was sprinkled, and the outer skin cut was closed with an automatic wound clip (Roboz, Reflex 7 Clip 7 mm). Following the operation, animals were kept in cages of no more than three animals whenever possible. After seven days, wound clips were removed. The sham surgery group was subjected to the same operation, and an equal volume of PBS was injected into the marrow cavity. The blank group was normally kept without any treatment. Administration was started 7 days after the modeling, and was performed once every 5 days and 3 times in total. Pain behaviors of mice were counted on day 14 (or day 15), and leg bone injury was scored on day 21.

Pain behavior counting method: Animals were placed on a wire mesh floor surrounded by organic glass and acclimatized for 30 min (until the animals stopped exploration and primary grooming activities in the box). Then their movements were observed, and behaviors of avoiding pain in the affected limb occurring within 5 min were assessed. The length of the behaviors was measured with a stopwatch.

Behaviors of avoiding pain are defined as follows:
(1) complete protection (walking with the affected limb lifted, standing without it on the ground);
(2) curling the digits of the affected limb and sticking through the mesh (A normal mouse will have the digits of its limbs stretched and spread on the mesh and stand on the anterior parts of its paws. A tumor bone metastasis mouse will have the digits of its affected limb curled. If it does so and can stand on the heels, the pain is deemed to be mild, and the behavior does not count. If the entire soles of its paws are not in contact with the mesh or stick through the mesh, the behavior counts);
(3) licking limbs on the affected side;
(4) sporadic single-leg jumps; and
(5) standing on the hind limb of one side (with both forelimbs lifted).

Bone injury scoring method:
The left femur in the prone position was carefully dissected and examined for bone destruction using an MX-20 digital cabinet X-ray system (Faxitron/Bioptics), and bone injury was scored by a designated professional.

The scoring criteria are as follows:
0-relatively normal, 1-mild-local injury, 2-moderate-local injury, 3-moderate-multiple injuries, 4-severe-diffuse injury. Scoring was performed on the distal end of the femur and the proximal end of the femur separately and the scores were added. Bone destruction scored 4 + 4 = 8 points when it was most severe.
1. Analgesic and bone-protecting effects of RANLK antagonists and NGF antagonists Since denosumab has no cross-binding activity for murine RANKL, we used the murine RANKL antibody AMR2 in combination with tanezumab to evaluate whether the combination of the two antibodies has better *in vivo* efficacy than one of them.

The variable region sequence of the antibody AMR2 is derived from WO2013176469A1. The variable region heavy and light chain sequences were fused to human IgG4 and human λ constant regions, respectively, to construct the full-length antibody AMR2. The related sequences of AMR2 are as follows:

**Table 14. The CDRs of the antibody AMR2**

| | | | |
|---|---|---|---|
| HCDR1 | DYDMS (SEQ ID NO: 102) | LCDR1 | TGSSSNIGNNAVS (SEQ ID NO: 105) |
| HCDR2 | WIYPSGGSIYYADSVKG (SEQ ID NO: 103) | LCDR2 | SDRHRPS (SEQ ID NO: 106) |
| HCDR3 | SGLTRTRWPIYYADGMDV (SEQ ID NO: 104) | LCDR3 | GSWDASLSGYV (SEQ ID NO: 107) |

AMR2 heavy chain variable region: 
VQLLESGGGLVQPGGSLRLSCAASGFTFSDYDMSWVRQAPGKGLEWVSWIYP
SGGSIYYADSVKGRFTISRDNSKNTLYLQMNSLRAEDTAVYYCARSGLTRTRWP
IYYADGMDVWGQGTLVTVSS
SEQ ID NO: 108;
AMR2 light chain variable region: 
QSVLTQPPSASGTPGQRVTISCTGSSSNIGNNAVSWYQQLPGTAPKLLIYSDRHR
PSGVPDRFSGSKSGTSASLAISGLRSEDEADYYCGSWDASLSGYVFGGGTKLT
VL
SEQ ID NO: 109;
AMR2 heavy chain 
EVQLLESGGGLVQPGGSLRLSCAASGFTFSDYDMSWVRQAPGKGLEWVSWIY
PSGGSIYYADSVKGRFTISRDNSKNTLYLQMNSLRAEDTAVYYCARSGLTRTRW
PIYYADGMDVWGQGTLVTVSSASTKGPSVFPLAPCSRSTSESTAALGCLVKDY
FPEPVTVSWNSGALTSGVHTFPAVLQSSGLYSLSSVVTVPSSSLGTKTYTCNVD
HKPSNTKVDKRVESKYGPPCPPCPAPEFLGGPSVFLFPPKPKDTLMISRTPEVTC
VVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSL
TCLVKGFYPSDIAVEWESNGQPENNYKTTPPVLDSDGSFFLYSRLTVDKSRWQE
GNVFSCSVMHEALHNHYTQKSLSLSLGK
SEQ ID NO: 110;
AMR2 light chain

QSVLTQPPSASGTPGQRVTISCTGSSSNIGNNAVSWYQQLPGTAPKLLIYSDRHR
PSGVPDRFSGSKSGTSASLAISGLRSEDEADYYCGSWDASLSGYVFGGGTKLT
VLGQPKANPTVTLFPPSSEELQANKATLVCLISDFYPGAVTVAWKADGSPVKA
GVETTKPSKQSNNKYAASSYLSLTPEQWKSHRSYSCQVTHEGSTVEKTVAPTE
CS
SEQ ID NO: 111.

Grouping and dosing are shown in Table 15:

**Table 15. Grouping and dosing**

| Group | Number of mice | Antibody | Dose (mpk) | Frequency of administration | Route of administration |
|---|---|---|---|---|---|
| 1 | 12 | Vehicle | / | Q5d*3 | i.p. |
| 2 | 12 | Tanezumab | 10 | Q5d*3 | i.p. |
| 3 | 12 | AMR2 | 20 | Q5d*3 | i.p. |
| 4 | 12 | Tanezumab + AMR2 | 10 + 20 | Q5d*3 | i.p. |
| Sham | 10 | / | / | / | / |
| Blank | 10 | / | / | / | / |

| | | | | | |
|---|---|---|---|---|---|
| Notes: Q5d*3 indicates that the administration was performed once every 5 days and 3 times in total; i.p. indicates intraperitoneal administration. | | | | | |

The experimental results are shown in FIG. 2A to FIG. 2D.

The results of counting pain behaviors of mice show that on day 14, three administration groups showed significant analgesic effects compared to the negative group, and the analgesic effect of the combination administration group was stronger than those of the mAb groups. On day 21, only the combination administration group showed a significant analgesic effect. Although the two mAb groups showed certain analgesic effects, but their effects were not statistically different from that of the negative group (vs vehicle: *P < 0.05, **P < 0.01, ***P < 0.0001).

The results of bone injury scoring show that the combination administration group exhibited a certain bone-protecting effect on day 14. The combination administration group showed a significant bone-protecting effect on day 21. The above experimental results show that the combination administration group demonstrated efficacy in both pain relieving and bone protection. Thus, the inventors further verified the *in vivo* efficacy of the bispecific antibody molecules of the present disclosure.

### 2. Evaluation of in vivo efficacy of the NGF terminus of the bispecific antibody

As the *in vitro* activity of the NGF terminus of bispecific antibody 1 is about 4 times lower than the NGF mAb tanezumab, whether the NGF terminus of bispecific antibody 1 is still effective in mice was determined by this efficacy experiment. Grouping is shown in the table below:

**Table 16. Dosing and grouping**

| Group | Number of mice | Drug | Dose (mpk) | Frequency of administration | Route of administration |
|---|---|---|---|---|---|
| 1 | 12 | Vehicle | / | Q5d | i.p. |
| 2 | 12 | Bispecific antibody 1 | 13.5 | Q5d | i.p. |

| | | | | | |
|---|---|---|---|---|---|
| Notes: Q5d indicates that the administration was performed once every 5 days; i.p. indicates intraperitoneal administration. | | | | | |

The experimental results are shown in FIG. 3A to FIG. 3B.

The results show that bispecific antibody 1 exhibited a significant analgesic effect compared to the negative group on both day 14 and day 21 after administration, which indicates that the NGF terminus of bispecific antibody 1 is still significantly effective.

### 3. Evaluation of in vivo efficacy of the bispecific antibody

An animal model of bone metastasis was constructed using human RANKL transgenic mice (purchased from Biocytogen) to evaluate the *in vivo* efficacy of the bispecific antibody. Grouping is shown in Table 17:

**Table 17. Dosing and grouping**

| Group | Number of mice | Drug | Dose (mpk) | Frequency of administration | Route of administration |
|---|---|---|---|---|---|
| 1 | 10 | Sham | | Q5d | i.p. |
| 2 | 10 | Vehicle | - | Q5d | i.p. |
| 3 | 14 | Bispecific | 5.4 | Q5d | i.p. |
| 4 | 14 | antibody 1 | 21.6 | Q5d | i.p. |

| | | | | | |
|---|---|---|---|---|---|
| Notes: Q5d indicates that the administration was performed once every 5 days; i.p. indicates intraperitoneal administration. | | | | | |

The experimental results are shown in FIG. 4A to FIG. 4D.

The results show that bispecific antibody 1 exhibited significant analgesic and bone-protecting effects. On both day 15 and day 21, the high-dose group of bispecific antibody 1 exhibited a significant analgesic effect, and both the high-dose and low-dose groups exhibited significant protective effects against bone injury.

### Test Example 9: PK Assays in Rats

An *in vivo* pharmacokinetic study was performed in SD rats. Male SD rats (Zhejiang Vital River Laboratory Animal Technology Co., Ltd.) were divided into groups of 4 and intravenously injected with bispecific antibody 1 at a dose of 4 mg/kg. Blood samples of 0.2 mL were collected from the administration groups before administration and 5 min, 8 h, 24 h, 48 h, 84 h, 9 d, 10 d, 14 d, 21 d, and 28 d after administration, and no anticoagulant was added. The blood samples were left at 4 °C for 30 min and centrifuged at 1000 g for 15 min. The serum in the upper layers was collected and placed into EP tubes and stored at -80 °C.

Serum concentration was determined by ELISA, and the pharmacokinetic parameters were calculated for the test antibody by Winnolin software. The results are shown below:

**Table 18. PK results for bispecific antibody 1**

| Test | RANKL arm | NGF arm |
|---|---|---|
| t1/2 (days) | 15.09 | 14.30 |

The results show that bispecific antibody 1 pharmacokinetically performed well in rats; the half-lives of the RANKL and NGF termini are 15.09 days and 14.3 days, respectively, which are close.

## Claims

1. An antigen-binding molecule, comprising:
at least one first antigen-binding domain that specifically binds to RANKL, and
at least one second antigen-binding domain that specifically binds to NGF;
the first antigen-binding domain that specifically binds to RANKL comprises a heavy chain variable region and a light chain variable region, wherein:
i) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
ii) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 15; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
iii) the heavy chain variable region comprises: a HCDR1 comprising the amino acid sequence of SEQ ID NO: 13; a HCDR2 comprising the amino acid sequence of SEQ ID NO: 8; and a HCDR3 comprising the amino acid sequence of SEQ ID NO: 3; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6.

2. The antigen-binding molecule according to claim 1, wherein the first antigen-binding domain that specifically binds to RANKL comprises a heavy chain variable region and a light chain variable region, wherein:
the heavy chain variable region comprises an amino acid sequence having at least 90% sequence identity to SEQ ID NO: 37, 36 or 38; and
the light chain variable region comprises an amino acid sequence having at least 90% sequence identity to SEQ ID NO: 40;
preferably,
the heavy chain variable region comprises the amino acid sequence of SEQ ID NO: 37, 36 or 38; and/or
the light chain variable region comprises the amino acid sequence of SEQ ID NO: 40;
more preferably,
the heavy chain variable region comprises the amino acid sequence of SEQ ID NO: 37; and/or
the light chain variable region comprises the amino acid sequence of SEQ ID NO: 40.

3. The antigen-binding molecule according to claim 1 or 2, wherein the second antigen-binding domain that specifically binds to NGF comprises a heavy chain variable region and a light chain variable region, wherein:
the heavy chain variable region comprises: a HCDR1 comprising the amino acid sequence of SEQ ID NO: 58; a HCDR2 comprising the amino acid sequence of SEQ ID NO: 59; and a HCDR3 comprising the amino acid sequence of SEQ ID NO: 60; and the light chain variable region comprises: a LCDR1 comprising the amino acid sequence of SEQ ID NO: 61; a LCDR2 comprising the amino acid sequence of SEQ ID NO: 62; and a LCDR3 comprising the amino acid sequence of SEQ ID NO: 63.

4. The antigen-binding molecule according to claim 3, wherein the second antigen-binding domain that specifically binds to NGF comprises a heavy chain variable region and a light chain variable region, wherein:
the heavy chain variable region comprises an amino acid sequence having at least 90% sequence identity to SEQ ID NO: 64; and/or
the light chain variable region comprises an amino acid sequence having at least 90% sequence identity to SEQ ID NO: 65;
preferably,
the heavy chain variable region comprises the amino acid sequence of SEQ ID NO: 64; and/or
the light chain variable region comprises the amino acid sequence of SEQ ID NO: 65.

5. The antigen-binding molecule according to any one of claims 1 to 4, comprising an Fc region, wherein the Fc region comprises two subunits capable of associating;
the Fc region is preferably an IgG₄ Fc region;
more preferably, the Fc region is a human IgG₄ Fc region, and the amino acid residue at position 228 is P, as numbered according to the EU index.

6. The antigen-binding molecule according to any one of claims 1 to 5, comprising a first chain of the structure of formula (I) and a second chain of the structure of formula (II),
VHa-linker 1-VHb-CHl-one subunit of Fc region formula (I);
VLa-linker 2-VLb-CL formula (II);
wherein:
VHa and VHb are heavy chain variable regions, and VLa and VLb are light chain variable regions;
VHa and VLa form the first antigen-binding domain that specifically binds to RANKL, and VHb and VLb form the second antigen-binding domain that specifically binds to NGF; or
VHa and VLa form the second antigen-binding domain that specifically binds to NGF, and VHb and VLb form the first antigen-binding domain that specifically binds to RANKL; and
linker 1 and linker 2 are peptide linkers identical or different in sequence;
preferably,
the VHa comprises the amino acid sequence of SEQ ID NO: 37, 36 or 38, and the VLa comprises the amino acid sequence of SEQ ID NO: 40; and/or
the VHb comprises the amino acid sequence of SEQ ID NO: 64, and the VLb comprises the amino acid sequence of SEQ ID NO: 65.

7. The antigen-binding molecule according to claim 6, wherein the linker 1 or linker 2 comprises the amino acid sequence set forth in any one selected from the group consisting of SEQ ID NOs: 70-97;
preferably, the linker 1 comprises the amino acid sequence of SEQ ID NO: 70, and/or the linker 2 comprises the amino acid sequence of SEQ ID NO: 85.

8. The antigen-binding molecule according to claim 6 or 7, wherein the CH1-Fc region comprises the amino acid sequence of SEQ ID NO: 51 or 66.

9. The antigen-binding molecule according to any one of claims 6 to 8, comprising:
a first chain, comprising an amino acid sequence having at least 85% sequence identity to SEQ ID NO: 98, 100 or 101; and/or
a second chain, comprising an amino acid sequence having at least 85% sequence identity to SEQ ID NO: 99;
preferably, the antigen-binding molecule comprises:
a first chain, comprising the amino acid sequence of SEQ ID NO: 98, 100 or 101; and
a second chain, comprising the amino acid sequence of SEQ ID NO: 99; more preferably, the antigen-binding molecule comprises two first chains identical in sequence and two second chains identical in sequence.

10. The antigen-binding molecule according to any one of claims 1 to 9, wherein the antigen-binding molecule has at least one of the following properties:
i) an affinity of the first antigen-binding domain that specifically binds to RANKL for RANKL is higher than an affinity of the second antigen-binding domain that specifically binds to NGF for NGF;
ii) an EC50 value with which the first antigen-binding domain that specifically binds to RANKL binds to human RANKL is less than an EC50 value with which the second antigen-binding domain that specifically binds to NGF binds to human NGF; and
iii) an IC50 value with which the first antigen-binding domain that specifically binds to RANKL blocks binding of RANKL to RANK is less than an IC50 value with which the second antigen-binding domain that specifically binds to NGF blocks binding of NGF to TrKA.

11. An anti-RANKL antibody, comprising a heavy chain variable region and a light chain variable region, wherein:
i) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 14; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
ii) the heavy chain variable region comprises: a HCDR1 comprising the amino acid sequence of SEQ ID NO: 7; a HCDR2 comprising the amino acid sequence of SEQ ID NO: 8; and a HCDR3 comprising the amino acid sequence of SEQ ID NO: 15; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6; or
iii) the heavy chain variable region comprises: a HCDR1 comprising the amino acid sequence of SEQ ID NO: 13; a HCDR2 comprising the amino acid sequence of SEQ ID NO: 8; and a HCDR3 comprising the amino acid sequence of SEQ ID NO: 3; and
the light chain variable region comprises: a LCDR1 comprising the amino acid sequence of SEQ ID NO: 4; a LCDR2 comprising the amino acid sequence of SEQ ID NO: 5; and a LCDR3 comprising the amino acid sequence of SEQ ID NO: 6.

12. The anti-RANKL antibody according to claim 11, wherein:
the heavy chain variable region comprises an amino acid sequence having at least 90% sequence identity to SEQ ID NO: 36, 37 or 38; and/or
the light chain variable region comprises an amino acid sequence having at least 90% sequence identity to SEQ ID NO: 40;
preferably, the heavy chain variable region comprises the amino acid sequence of SEQ ID NO: 36, 37 or 38; and/or the light chain variable region comprises the amino acid sequence of SEQ ID NO: 40;
more preferably, the heavy chain variable region comprises the amino acid sequence of SEQ ID NO: 37; and/or the light chain variable region comprises the amino acid sequence of SEQ ID NO: 40.

13. The anti-RANKL antibody according to claim 11 or 12, comprising a heavy chain constant region and a light chain constant region, wherein preferably, the heavy chain constant region comprises the amino acid sequence of SEQ ID NO: 51 or 66, and/or the light chain constant region comprises the amino acid sequence of SEQ ID NO: 52.

14. The anti-RANKL antibody according to any one of claims 11 to 13, comprising a heavy chain and a light chain, wherein:
the heavy chain comprises an amino acid sequence having at least 85% sequence identity to SEQ ID NO: 53, 55 or 56, and/or the light chain comprises an amino acid sequence having at least 85% sequence identity to SEQ ID NO: 54;
preferably,
the heavy chain comprises the amino acid sequence of SEQ ID NO: 53, and/or the light chain comprises the amino acid sequence of SEQ ID NO: 54; or
the heavy chain comprises the amino acid sequence of SEQ ID NO: 55, and/or the light chain comprises the amino acid sequence of SEQ ID NO: 54; or
the heavy chain comprises the amino acid sequence of SEQ ID NO: 56, and/or the light chain comprises the amino acid sequence of SEQ ID NO: 54.

15. The anti-RANKL antibody according to any one of claims 11 to 13, wherein the anti-RANKL antibody is an antibody fragment; preferably, the antibody fragment is selected from the group consisting of Fab, Fab', F(ab')2, Fd, Fv, scFv, dsFv and dAb.

16. The anti-RANKL antibody according to any one of claims 11 to 15, wherein:
the antibody binds to human RANKL with a KD value of less than 5 nM; and/or
the antibody blocks binding of RANKL to RANK with an IC50 value of less than 0.5 nM.

17. A bispecific antibody, comprising the anti-RANKL antibody according to any one of claims 11 to 16.

18. An isolated nucleic acid, encoding the antigen-binding molecule according to any one of claims 1 to 10, or the anti-RANKL antibody according to any one of claims 11 to 16, or the bispecific antibody according to claim 17.

19. A vector, comprising the isolated nucleic acid according to claim 18.

20. A host cell, comprising the vector according to claim 19.

21. A method for preparing an antigen-binding molecule, an anti-RANKL antibody or a bispecific antibody, comprising culturing the host cell according to claim 20 under conditions suitable for expression of the antigen-binding molecule or antibody.

22. A pharmaceutical composition, comprising:
a therapeutically effective amount of the antigen-binding molecule according to any one of claims 1 to 10, or the anti-RANKL antibody according to any one of claims 11 to 16,
or the bispecific antibody according to claim 17; and
one or more pharmaceutically acceptable carriers, diluents, buffers or excipients.

23. A method for treating or preventing a disease, wherein the method comprises administering to a subject a therapeutically effective amount of the antigen-binding molecule according to any one of claims 1 to 10, or the anti-RANKL antibody according to any one of claims 11 to 16, or the bispecific antibody according to claim 17, or the pharmaceutical composition according to claim 22;
preferably, the disease is pain, joint stiffness or bone loss;
more preferably, the pain is selected from the group consisting of osteoarticular pain, rheumatoid arthritis pain, gout, bone cancer pain, fracture pain, post-surgical pain, cancer pain, painful bladder syndrome, musculoskeletal pain, prostatitis, pelvic pain, interstitial cystitis, lower back pain, dysmenorrhea, pain associated with bone disease, trigeminal neuralgia, post-herpetic neuralgia, herpes zoster infection, sciatica, migraine, diabetic neuropathy, and pain associated with peripheral nerves;
wherein the bone loss is associated with at least one condition selected from the group consisting of osteoporosis, Paget's disease, osteomyelitis, hypercalcemia, osteopenia, osteoporosis, osteonecrosis, bone injury, bone resorption, osteogenesis imperfecta, inflammation, autoimmune disease, enteritis, rheumatoid arthritis, systemic lupus erythematosus, Crohn's disease, periodontal bone resorption, osteolytic metastasis, and cancer.

24. The method according to claim 23, wherein the cancer is selected from the group consisting of breast cancer, prostate cancer, thyroid cancer, kidney cancer, lung cancer, esophageal cancer, rectal cancer, bladder cancer, cervical cancer, ovarian cancer, liver cancer, gastrointestinal cancer, melanoma, multiple myeloma, osteosarcoma, lymphoma, non-small cell lung cancer, bone tumor, and Hodgkin's disease.

25. A method for treating or preventing an NGF- or RANKL-associated disease, wherein the method comprises administering to a subject a prophylactically or therapeutically effective amount of the antigen-binding molecule according to any one of claims 1 to 10, or the anti-RANKL antibody according to any one of claims 11 to 16, or the bispecific antibody according to claim 17, or the pharmaceutical composition according to claim 22;
preferably, the NGF- or RANKL-associated disease is pain, joint stiffness or bone loss; more preferably, the pain is selected from the group consisting of osteoarticular pain, rheumatoid arthritis pain, gout, bone cancer pain, fracture pain, post-surgical pain, cancer pain, painful bladder syndrome, musculoskeletal pain, prostatitis, pelvic pain, interstitial cystitis, lower back pain, dysmenorrhea, pain associated with bone disease, trigeminal neuralgia, post-herpetic neuralgia, herpes zoster infection, sciatica, migraine, diabetic neuropathy, and pain associated with peripheral nerves;
wherein the bone loss is associated with at least one condition selected from the group consisting of osteoporosis, Paget's disease, osteomyelitis, hypercalcemia, osteopenia, osteoporosis, osteonecrosis, bone injury, bone resorption, osteogenesis imperfecta, inflammation, autoimmune disease, enteritis, rheumatoid arthritis, systemic lupus erythematosus, Crohn's disease, periodontal bone resorption, osteolytic metastasis, and cancer.

26. A method for treating pain or inhibiting bone loss, wherein the method comprises administering to an individual in need thereof an effective amount of an NGF antagonist and a RANKL antagonist; the NGF antagonist and the RANKL antagonist are administered simultaneously or sequentially;
preferably, the NGF antagonist is an anti-NGF antibody and/or the RANKL antagonist is an anti-RANKL antibody.

27. The method according to claim 26, wherein:
the anti-NGF antibody is tanezumab, and/or
the anti-RANKL antibody is denosumab.
